(19) Europäisches Patentamt
European Patent Office
Office européen des brevets

(11) Publication number: 0 005 014 A1

# (12) EUROPEAN PATENT APPLICATION

(21) Application number: 79300515.8

(22) Date of filing: 29.03.79

(51) Int. Cl.[2]: C 07 D 498/04
A 61 K 31/42

(30) Priority: 22.04.78 GB 1598578
09.09.78 GB 3626778
27.10.78 GB 4233878
11.10.78 GB 4014878

(43) Date of publication of application:
31.10.79 Bulletin 79/22

(84) Designated Contracting States:
BE CH DE FR GB IT LU NL SE

(71) Applicant: BEECHAM GROUP LIMITED
Beecham House Great West Road
Brentford, Middlesex(GB)

(72) Inventor: Stirling, Irene
80 Woodcrest Walk
Reigate Surrey(GB)

(72) Inventor: Clarke, Brian Peter
"Coneybury" Drive Spur Forest Drive
Kingswood Surrey(GB)

(72) Inventor: Harbridge, John Barry
109 St. Andrews Road
Coulsdon Surrey(GB)

(74) Representative: Cole, William Gwyn et al,
Beecham Pharmaceuticals Yew Tree Bottom Road
Epsom Surrey KT18 5XQ(GB)

## (54) Clavulanic acid derivatives, preparation and use.

(57) This invention provides the β-lactamase inhibitory compounds of the formula (II):

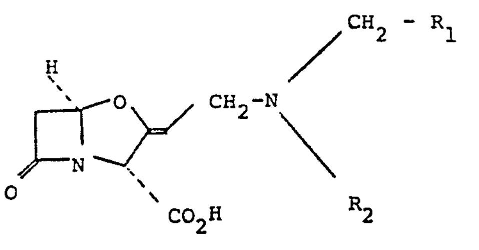

(II)

and esters and acid addition salts of esters thereof wherein $R_1$ is an optionally substituted aromatic monocyclic or bicyclic heterocyclic group bonded via a carbon atom and $R_2$ is a group $CH_2R_1$, a hydrogen atom, a $C_{1-6}$ alkyl or hydroxyalkyl group, an optionally substituted benzyl group, or a group of the sub-formula (a):

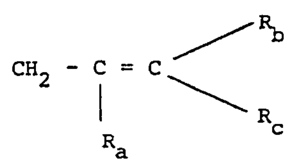

(a)

where $R_a$ is a $C_{1-3}$ alkyl group, and $R_b$ and $R_c$ are independently hydrogen, $C_{1-3}$ alkyl or phenyl.

The invention also relates to processes for their preparation and pharmaceutical compositions containing them.

EP 0 005 014 A1

# Clavulanic Acid Derivatives, Preparation and Use

The present invention relates to β-lactamase inhibitory agents, to a process for their preparation and to compositions containing them.

British Patent Application No: 41887/75 (see also U.S. Serial No: 731928, Belgian Patent No: 847044, West German Offenlegungsschrift No: P 2646003.7) discloses *inter alia* the compounds of the formula (I):

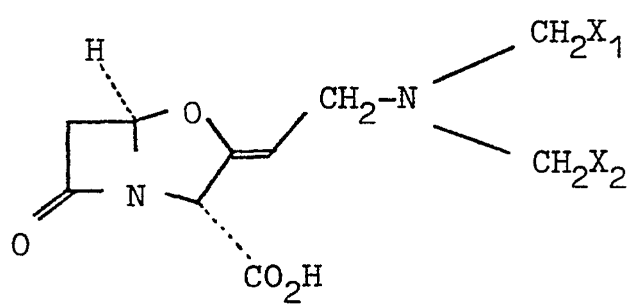

(I)

and esters thereof wherein $X_1$ is hydrogen atom, an alkyl group of up to 5 carbon atoms, an alkenyl group of up to 5 carbon atoms, a hydroxy alkyl group of up to 5 carbon atoms or an optionally substituted phenyl group and $X_2$ is an optionally substituted phenyl group. Such compounds were described as antibacterial agents and β-lactamase

inhibitors.

We have now discovered that certain novel amines are β-lactamase inhibitors which enhance the effectiveness of penicillins and cephalosporins, and which have anti-bacterial activity.

The present invention provides the compounds of the formula (II) :

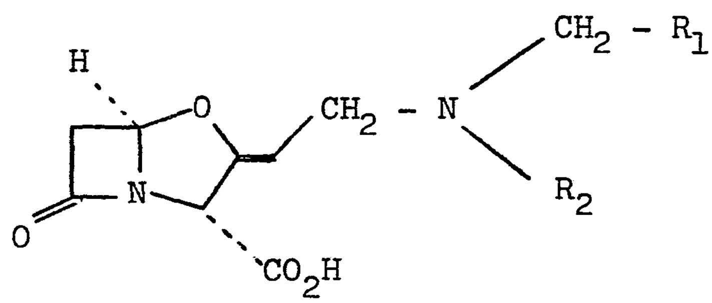

(II)

and esters and acid addition salts of esters thereof wherein $R_1$ is an optionally substituted aromatic monocyclic or bicyclic heterocyclic group bonded via a carbon atom and $R_2$ is a group $CH_2R_1$, a hydrogen atom, a $C_{1-6}$ alkyl or hydroxyalkyl group, an optionally substituted benzyl group, or a group of the sub-formula (a) :

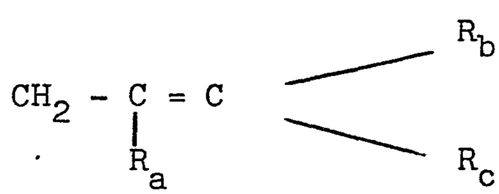

(a)

where $R_a$ is a $C_{1-3}$ alkyl group, and $R_b$ and $R_c$ are independently hydrogen, $C_{1-3}$ alkyl or phenyl.

Suitable aromatic monocyclic heterocyclic groups are those containing 5 or 6 ring atoms.

Suitable aromatic bicyclic heterocyclic groups are those having a benzene ring fused to an aromatic heterocyclic group containing 5 or 6 ring atoms.

Suitable groups $R_1$ may be represented by the sub-formula (i) :

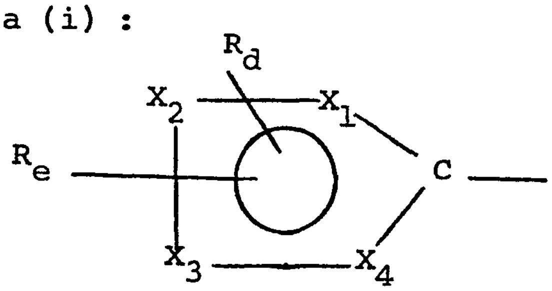

(i)

wherein one of $X_1$, $X_2$, $X_3$ and $X_4$ is -CHN-, -O-, -S- or $-NR_f-$ where $R_f$ is H or lower alkyl, and the others are selected from -N- and -CH-; $R_d$ is hydrogen; and $R_e$ is hydrogen, lower alkyl, CN, $CO_2H$, esterified $CO_2H$, $NO_2$ or the like; or $R_d$ and $R_e$ together form the residue of an optionally substituted benzene ring.

When used with reference to the substituents in the group (i), "esterified carboxyl" means a carboxyl group esterified by a $C_{1-6}$ alkyl or $C_{1-6}$ alkoxy-$C_{1-6}$ alkyl group or a benzyl group optionally substituted by a nitro, $C_{1-4}$ alkyl or $C_{1-4}$ alkoxy group or a fluorine, chlorine or bromine atom.

Suitable heterocyclic groups are those which contain up to three heteroatoms in the ring system.

Suitable heteroatoms in the group $R_1$ are oxygen, sulphur and nitrogen.

Normally not more than one oxygen or one sulphur atom will be present in the ring.

When used herein the term "optionally substituted" means unsubstituted or substituted by up to three atoms or groups selected from fluorine, chlorine, bromine, alkyl, alkoxy or acyloxy of 1-3 carbon atoms, nitro, nitrilo, hydroxyl or the like.

Preferably, the group $R_1$ is unsubstituted or is substituted by a lower alkyl, $NO_2$, CN, $CO_2H$, esterified $CO_2H$ or the like group.

Thus, suitable groups $R_1$ include the furyl, thienyl, pyrrolyl, N-methylpyrrolyl, pyridyl, indolyl, oxazolyl, thiazolyl, quinolyl, and the like groups.

Suitably, $R_2$ is a hydrogen atom or a methyl, ethyl, propyl, butyl, hydroxymethyl, hydroxyethyl or hydroxypropyl group.

A particularly preferred group of compounds of this invention are those wherein $R_2$ is a hydrogen atom, since they have particularly good β-lactamase inhibitory and antibacterial effectiveness.

A further suitable group of compounds of the invention are those wherein $R_2$ is a group of the sub-formula (b) :

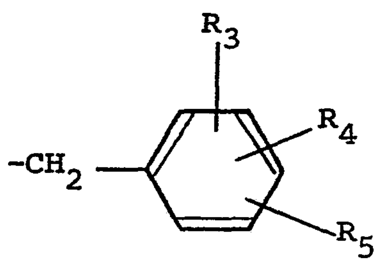

(b)

wherein $R_3$ is a hydrogen, fluorine, chlorine or bromine atom or an alkyl group of 1-3 carbon atoms, an alkoxyl group of 1-3 carbon atoms, an acyloxy group of 1-3 carbon atoms, a hydroxyl group or an alkoxycarbonyl group containing 1-3 carbon atoms in the alkoxy part; $R_4$ is a hydrogen, fluorine or chlorine atom or an alkyl group of 1-3 carbon atoms, an alkoxy group of 1-3 carbon atoms or an acyloxy group of 1-3 carbon atoms; $R_5$ is a hydrogen, fluorine or chlorine atom or an alkyl group of 1-3 carbon atoms or an alkoxyl group of 1-3 carbon atoms.

Suitable groups of the sub-formula (b) include the benzyl, p-methylbenzyl, p-methoxybenzyl, p-fluorobenzyl and p-chlorobenzyl groups. Of these groups, the benzyl group is preferred, since compounds (II) where $R_2$ is benzyl are favoured intermediates.

Preferred groups (a) are the 2-methylallyl and 2-methyl-3-phenylallyl groups.

A further useful group of the compounds of the invention are those wherein $R_2$ is a group $CH_2R_1$.

A further preferred group of compounds are those of the formula (III) :

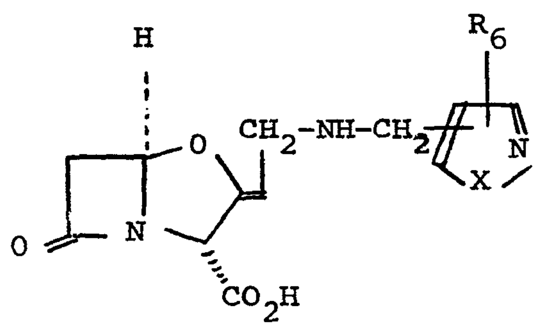

(III)

wherein X is a sulphur atom or an oxygen atom and $R_6$ is a hydrogen atom or an alkyl group of 1-3 carbon atoms.

Since the compounds of the formula (III) exist as zwitterions, they may also be represented by the formulae (IV), (V), (VI), (VII), (XVI) or (XVII) :

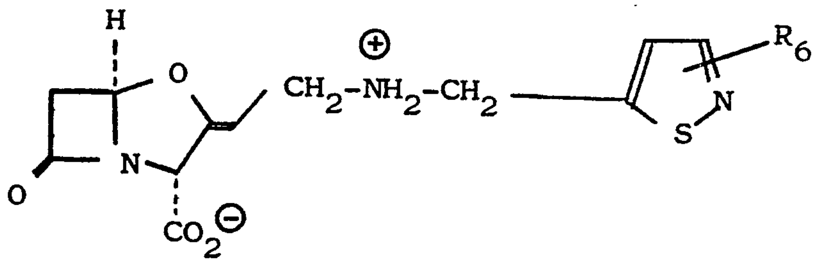

(IV)

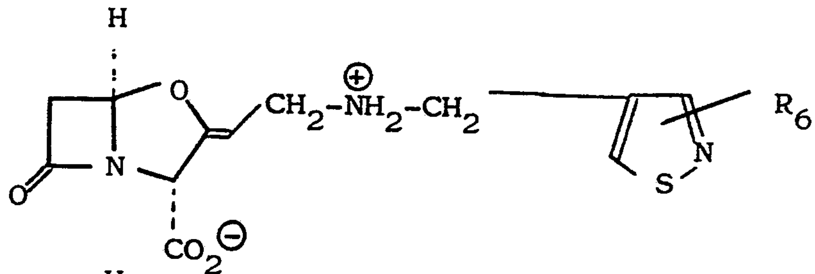

(V)

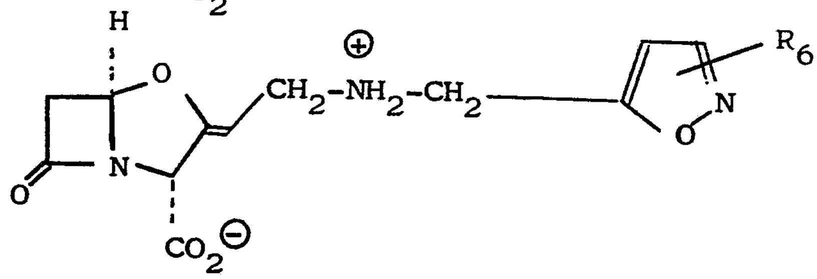

(VI)

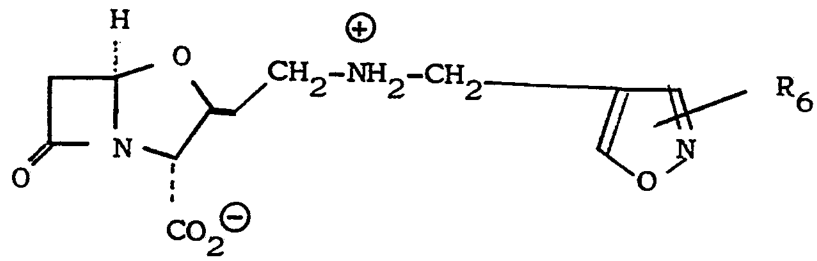

(VII)

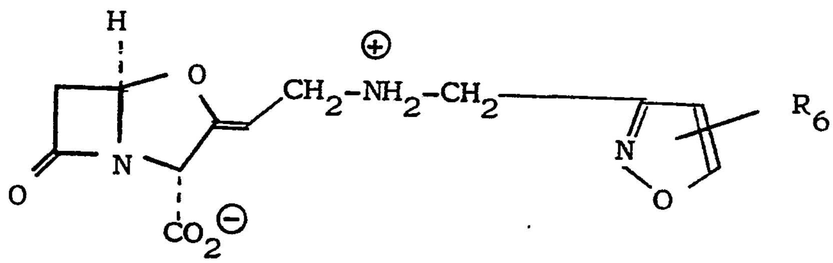

(XVI)

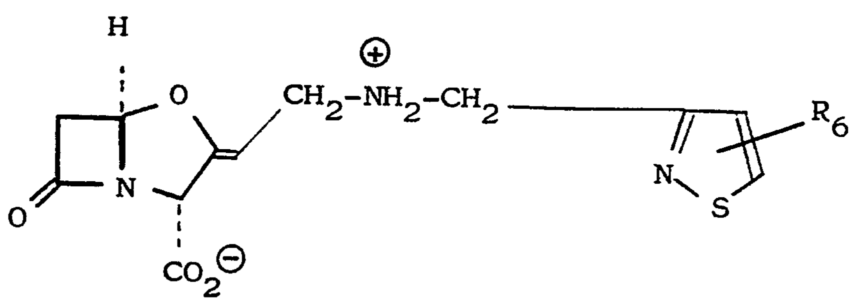

(XVII)

wherein $R_6$ is as defined in relation to formula (III).

Favourably $R_6$ is a hydrogen atom or a methyl group. Aptly the compound of this invention is of the formula (IV) or (V). Aptly the compound of this invention is of the formula (VI) or (VII); (XVI) or (XVII).

Certain suitable esters of the compounds of the formula (II) include those of the formulae (VIII) and (IX):

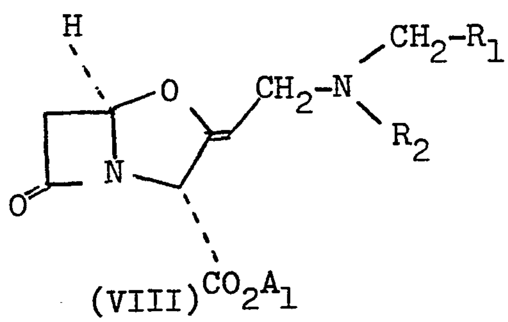

(VIII)

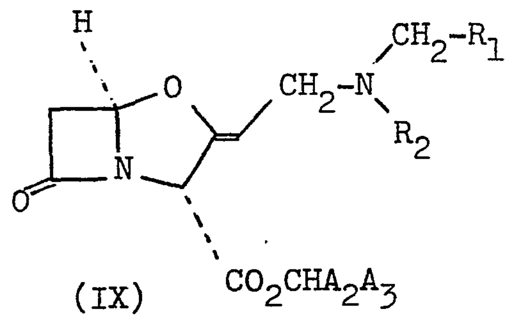

(IX)

wherein $A_1$ is an alkyl group of 1-6 carbon atoms optionally substituted by an alkoxyl or acyloxyl group of 1-7 carbon atoms; $A_2$ is an alkenyl group of up to 5 carbon atoms or is a phenyl group optionally substituted by a fluorine, chlorine, bromine, nitro or alkyl or alkoxyl of up to 4 carbon atoms; and $A_3$ is a hydrogen atom, an alkyl group of up to 4 carbon atoms or a phenyl group optionally substituted by a fluorine, chlorine, bromine, nitro or alkyl or alkoxyl of up to 4 carbon atoms.

Suitable esters of the compounds of the formula (II) include the methyl, ethyl, n-propyl, n-butyl, allyl, $CH_2C{\equiv}CH$, methoxy-methyl, acetoxymethyl, priopionoxymethyl, pivaloyloxymethyl, ethoxycarbonyloxymethyl, methoxycarbonyloxyethyl, ethoxycarbonyloxyethyl, dimethoxyphthalidyl, benzyl, methoxybenzyl, ethoxybenzyl, nitrobenzyl, chlorobenzyl or the like group.

Certain favoured groups $A_1$ include the methyl, methoxymethyl, acetoxymethyl, acetoxyethyl, phthalidyl, ethoxycarbonyloxy-methyl, α-ethoxycarbonyloxyethyl and the like groups.

Certain favoured groups $A_2$ include the phenyl p-nitrophenyl and 4-methoxyphenyl groups. A particularly favoured moiety $A_3$ is the hydrogen atom.

Suitable acid addition salts of esters of the compounds of the formula (II) are acid addition salts with pharmaceutically acceptable acids, such as hydrochloric, phosphoric, sulphuric, methanesulphonic, toluenesulphonic, citric, malic, acetic, lactic, tartaric, propionic, succinic and the like acids.

In general, esters are not provided in salted form.

Esters of the compounds of the formula (II) wherein $R_2$ is not a hydrogen atom tend to be more stable than those wherein $R_2$ is a hydrogen atom. Esters of the compounds of the formula (II) wherein $R_2$ is not a hydrogen atom are preferably those cleavable by hydrogenolysis, since they serve as intermediates to the corresponding zwitterionic compounds of the formula (II).

Most suitably, the compounds of this invention are in the form of the zwitterionic compound of the formula (II), especially those wherein $R_2$ is a hydrogen atom.

This invention also provides a process for the preparation of the compounds of the formula (II), which process comprises the reaction of an ester of a compound of the formula (X):

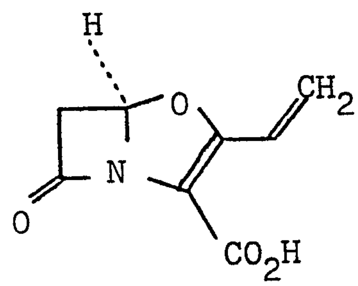

(X)

with a compound of the formula (XI):

$HN(R_2)CH_2R_1$ (XI)

wherein $R_1$ and $R_2$ are as defined in relation to formula (II), except that $R_2$ is not a hydrogen atom, and thereafter, if desired, performing one or more of the following reactions:

(a) when it is desired to form a compound (II) where $R_2$ is H, converting a compound where $R_2$ is a group (a), $CH_2R_1$ or benzyl to the corresponding compound where $R_2$ is H by hydrogenation;

(b) converting the ester of the compound of the formula (II) to an acid addition salt, the free acid or an alternative ester or acid addition salt thereof.

The ester of the compound of the formula (X) used in the process may be produced by the methods disclosed in British Patent Application NO: 41887/75, or equivalent disclosures referred to above. These methods include the reaction of a base with an esterified 9-O-acyl derivative of clavulanic acid, such as benzyl dichloroacetylclavulanate.

The conditions employed for the reaction of the compounds of the formulae (X) and (XI) will be as described for the analogous reaction disclosed in British Patent Application No: 41887/75, or equivalent disclosures.

The formation of a compound of the formula (II) wherein $R_2$ is a hydrogen atom may be achieved by hydrogenation of an ester of a compound of the formula (II) wherein $R_2$ is benzyl or a group (a) or $CH_2R_1$ after the reaction of the compounds of the formulae (X) and (XI) and thereafter, if desired, carrying out reaction (b) as hereinbefore described.

Preferred hydrogenolysable groups $R_2$ are benzyl, pyridylmethyl, 2-methyallyl or 2-methyl-3-phenylallyl.

The hydrogenation is generally carried out in the presence of a transition metal catalyst such as palladium, for example in the form of palladium on charcoal, at a low, medium or high pressure of hydrogen, for example from 1 to 6 atmospheres. The hydrogenation is generally performed in an inert solvent, such as ethanol, propanol, tetrahydrofuran, dioxane, ethyl acetate or a mixture of such solvents, optionally in the presence of water.

If the ester used in the hydrogenation is one which is cleaved by hydrogenation, the zwitterionic form of the secondary amine of the formula (II) is produced.

The present invention also provides a process for the preparation of a compound of the formula (II) wherein $R_2$ is hydrogen which process comprises the reduction with a water soluble complex hydride of a salt of a compound of the formula (XII):

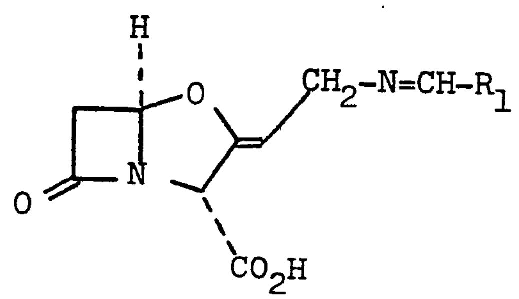

(XII)

wherein $R_1$ is as defined in relation to formula (II).

Suitable water soluble complex hydrides include borohydrides such as lithium borohydride, sodium borohydride, potassium borohydride, sodium cyanoborohydride or the like. In general an excess of the hydride is employed.

The reaction is carried out in an aqueous medium, for example in water or in a mixture of water with an inert water-miscible organic solvent such as tetrahydrofuran, dioxan or the like.

It is a favoured feature of this invention that ambient and near ambient temperatures may be employed, for example the reaction may be carried out at a temperature of $0^{o}$ to $30^{o}$ and conveniently at ambient, for example at about $18^{o}$ - $25^{o}$.

The pH of the reaction is best kept below 10 and this may be effected by the addition of an acid such as hydrochloric or like mineral acid simultaneously with the complex hydride. This may be carried out in a pH-stat or other similar system. (Metrohm, Herisau).

Once the reaction is over it is advantageous to return the pH to about 5 - 8.

The desired product may be obtained from the reaction mixture by evaporation of the solvent. Purification may be effected by crystallisation (for example before all the solvent has been evaporated off) or by column chromatography, for example using silica gel or cellulose and butanol/ethanol/water 4/4/1.

A preferred aspect of this process comprises the reduction of a compound of the formula (XIII):

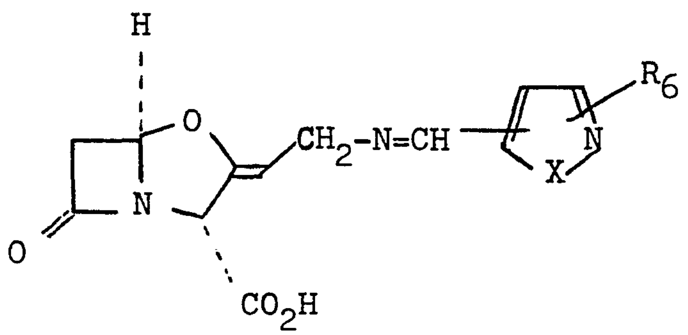

(XIII)

wherein X and $R_6$ are as defined in relation to formula (III).

The compounds of the formula (XII) and (XIII) are novel and as such form an aspect of this invention.

A process for the preparation of acid addition salts of esters of the compounds of the formula (II) wherein $R_2$ is a hydrogen atom comprises the hydrogenation of a corresponding ester of a compound of the formula (XIV):

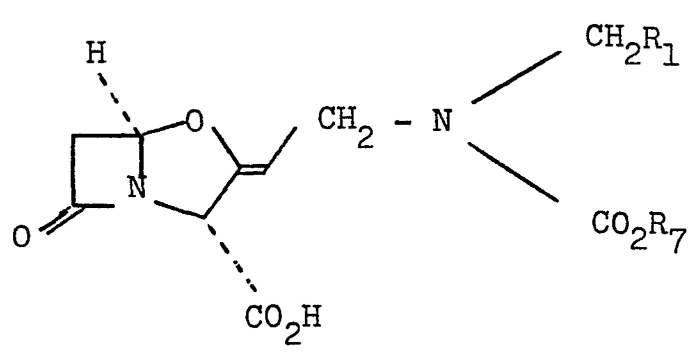

(XIV)

wherein $R_1$ is as defined in relation to formula (II) and $R_7$ is a group of the sub-formula (b) as defined in relation to formula (II), and thereafter converting the product to the acid addition salt.

Most suitably $R_7$ is a benzyl group.

Preferably the ester of the compound of the formula (XIV) is an ester as defined in relation to formula (VIII).

Particularly suitable esters of the compound of the formula (XIV) include $C_{1-4}$ alkyl esters, especially the methyl and ethyl esters.

The hydrogenation of the ester of the compound of the formula (XIV) will take place under reaction conditions similar to those described for the hydrogenation of an ester of a compound of the formula (II), but in the presence of acid or with the subsequent addition of acid to form the acid addition salt.

Esters of compounds of the formula (XIV) may be prepared by the reaction of a compound of the formula (II) wherein $R_2$ is hydrogen with, for example, a compound $Cl.CO\text{-}OCH_2R_6$, as described in German Patent Application No: P 2817085.6, followed by esterification in conventional manner. (See also U.S. Serial No: 896441).

The free acid or zwitterionic form of the compounds of the formula (II) may be produced by de-esterifying a corresponding ester of the compound of the formula (II). The preferred method of de-esterification is the hydrogenation of a hydrogenolysable ester. Suitable hydrogenolysable esters include the benzyl and substituted benzyl esters. Suitable substituted benzyl esters are methylbenzyl, methoxybenzyl, halobenzyl, nitrobenzyl and the like esters.

The conditions for this de-esterification reaction will be as hereinbefore described for hydrogenation to form a compound of the formula (II) wherein $R_2$ is hydrogen.

Compounds of the formula (II) may be prepared by the hydrolysis of a corresponding ester under mild basic conditions, for example, by the careful addition of base to a solution of the ester. Suitably, a pH-stat is used, the pH being maintained at about 8 to 8.5.

Particularly suitable esters for hydrolysis include the methyl ester and the methoxymethyl ester.

Esters of the compounds of the formula (II) may be converted to alternative esters by de-esterification as hereinbefore described, followed by re-esterification. Suitable esterifying agents for use in this process include reactive halides, alkyl oxonium salts, and the like. The re-esterification reaction will normally take place in a solvent, such as dimethylformamide, hexamethylphosphoramide, dichloromethane, ethyl acetate, or other non-esterifiable solvent, at a temperature of from about $0^o$ to about $25^o$.

Acid addition salts of esters of the compounds of the formula (II) may be prepared by the reaction of the unsalted ester with an acid, for example, in a dry organic solvent.

The present invention also provides a process for the preparation of a compound of the formula (XII) which process comprises the reaction of 9-aminodeoxyclavulanic acid with a compound of the formula (XV) :

$$R_1 - CHO \qquad (XV)$$

wherein $R_1$ is as defined in relation to formula (II), in an aqueous solvent wherein the solution is maintained at an alkaline pH.

The pH of the solution is most suitably maintained in the region of 7 - 10 and preferably 8 - 9. This may be effected by the addition of base such as an alkali or alkaline earth metal hydroxide, a carbonate or bicarbonate or with a strong organic base which is unreactive towards aldehydes. Thus suitable bases include lithium hydroxide, sodium hydroxide, potassium hydroxide, calcium hydroxide, barium hydroxide, sodium carbonate, potassium bicarbonate, triethylamine and the like. It is convenient to add the base automatically, for example in a pH-stat.

Solvents suitable for use in this process include water and water in a mixture with inert water miscible organic solvents such as tetrahydrofuran, dioxane, dimethylformamide and the like.

The temperature under which this reaction proceeds is convenient in that it is at or near ambient for example $0^o$ - $30^oC$ and more suitably $18^o$ - $25^oC$.

Most suitably an excess of the aldehyde is present, for example a 2 - 10 fold excess.

The compound of the formula (XII) is generally only stable in the presence of excess of the aldehyde For this reason, and for general convenience, it is preferred to form and use the compound of the formula (XII) in situ. This adds to the commercial attractiveness of this over-all process for the preparation of the compounds of the formula (II).

The present invention provides a pharmaceutical composition which comprises a compound of this invention and a pharmaceutically acceptable carrier.

The compositions of the invention include those in a form adapted for oral, topical or parenteral use and may be used for the treatment of the infection in mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating antibiotics.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form. The zwitterionic compounds of this invention are particularly suitable for use in such compositions.

The injectable solution of the compound of this invention may be made up in a sterile pyrogen-free liquid such as water, aqueous ethanol or the like.

Unit dose compositions comprising a compound of this invention adapted for oral administration form a further suitable composition aspect of this invention. However, orally administrable forms are generally less favoured than injectable forms owing to the relatively poorer absorption of the compound from the gastro-intestinal tract. Despite this, orally administrable compositions are of use as a synergistically effective

blood level can be expected at high doses and at lower doses such compositions may be used to treat infections localised in the gastro-intestinal tract.

Pharmaceutical compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compound of the formula may be present in the composition as sole therapeutic agent or it may be present together with other therapeutic agents such as a penicillin or cephalosporin. Considerable advantages accrue from the inclusion of a penicillin or cephalosporin since the resulting composition shows enhanced effectiveness (synergy).

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbencillin, azlocillin, mezlocillin, celbenicillin, and other known penicillins including pro-drugs therefore such as their in-vivo hydrolysable esters such as the acetoxymethyl, pivaloyloxymethyl, α-ethoxycarbonyloxyethyl or phthalidyl esters of ampicillin, benzylpenicillin or amoxycillin, and aldehyde or ketone adducts of penicillins containing a 6-α-aminoacetamido side chain (such as hetacillin, metampicillin and analogous derivatives of amoxycillin) or α-esters of carbenicillin or ticarcillin such as their phenyl or indanyl α-esters.

Suitable cephalosporins for inclusion in the compositions of this invention include cefatrizine,

cephaloridine, cephalothin, cefazolin, cephalexin, cephacetrile, cephamandole nafate, cephapirin, cepradine, 4-hydroxycephalexin, cefaparole, cephaloglycin, and other known cephalosporins or pro-drugs thereof.

Such compounds are frequently used in the form of a salt or hydrate or the like.

Naturally if the penicillin or cephalosporin present in the composition is not suitable for oral administration then the composition will be adapted for parenteral administration. As previously indicated, such injectable or infusable compositions are preferred.

Highly favoured penicillins for use in the compositions of this invention include ampicillin, amoxycillin, carbenicillin and ticarcillin. Such penicillins may be used as a pharmaceutically acceptable salt such as the sodium salt. Alternatively, the ampicillin or amoxycillin may be used in the form of fine particles of the zwitterionic form (generally as ampicillin trihydrate or amoxycillin trihydrate) for use in an injectable suspension, for example, in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin. Such cephalosporins may be used as a pharmaceutically acceptable salt, for example the sodium salt.

When present together with a cephalosporin or penicillin, the ratio of a compound of the invention to the penicillin or cephalosporin agent may vary over

a wide range of ratios, such as from 10:1 to 1:10 for example about 3:1, 2:1, 1:1, 1:2, 1:3, 1:4, 1:5, or 1:6, (wt/wt, based on pure free antibiotic equivalent). Orally administrable compositions containing a compound of the invention will normally contain relatively more synergist than corresponding injectable compositions, for example the ratio in an oral composition may be from about 3:1 to about 1:1, whereas a corresponding injectable composition may contain a ratio of about 1:1 to about 1:3 (compound of the invention:penicillin or cephalosporin.)

The total quantity of a compound of the invention in any unit dosage form will normally be between 25 and 1000 mg and will usually be between 50 and 500 mg, for example about 62.5, 100, 125, 150, 200 or 250 mg.

Compositions of this invention may be used for the treatment of infections of inter alia, the respiratory tract, the urinary tract and soft tissues in humans and mastitis in cattle.

Normally between 50 and 1000 mg of the compounds of the invention will be administered each day of treatment but more usually between 100 and 750 mg of the compounds of the invention will be administered per day, for example as 1-6 doses, more usually as 2, 3 or 4 doses.

The penicillin or cephalosporin in the synergistic composition of this invention will normally be present at approximately the amount at which it is conveniently used which will usually be expected to be from about 62.5 to 1000 mg per dose, more usually about 125, 250 or 500 mg per dose.

One particularly favoured composition of this invention will contain from 150 to 1000 mg of amoxycillin as the trihydrate or sodium salt and from 25 to 500 mg of a compound of this invention.

A further particularly favoured composition of this invention will contain from 150 to 1000 mg of ampicillin or a pro-drug therefor and from 25 to 500 mg of a compound of this invention.

Most suitably this form of composition will contain ampicillin trihydrate, ampicillin anhydrate, sodium ampicillin, hetacillin, pivampicillinhydrochloride, bacampicillin hydrochloride, or talampicillin hydrochloride.

Most suitably the preceding compositions will contain from 200 to 700 mg of the penicillin component. Most suitably the preceding composition will comprise from 50 to 250 mg of a compound of the invention.

Such compositions may be adapted for oral or parenteral use except when containing an in-vivo hydrolysable ester of ampicillin or amoxycillin in which case the compositions will not be adapted for parenteral administration.

Another particularly favoured composition of this invention will contain from 200 to 2000 mg of carbenicillin, ticarcillin or a pro-drug therefor and from 50 to 500 mg of a compound of the invention.

Suitably this form of composition will contain di-sodium carbenicillin. Suitably this form of the composition will contain di-sodium ticarcillin.

More suitably this form of the composition will contain from 75 to 250 mg of a compound of the invention. Such compositions containing di-salts of carbenicillin and ticarcillin will be adapted for parenteral administration.

The present invention also provides a method of treating bacterial infections in humans or domestic mammals which comprises the administration of a composition of this invention.

Commonly the infection treated will be due to a strain of Staphylococcus aureus, Klebsiella aerogenes, Escherichia coli, Proteus sp. or the like. The organism believed to be most readily treated by an antibacterially effective amount of amount of a compound of this invention is Staphylococcus aureus. The other organisms named are more readily treated by using a synergistically effective amount of the compound of the invention and a penicillin or cephalosporin. The administration of the two components may take place separately but in general we prefer to use a composition containing both the synergist and the penicillin or cephalosporin.

The indications for treatment include respiratory tract and urinary tract infections in humans and mastitis in cattle.

The following Examples illustrate the invention:

## Description 1

## p-Nitrobenzyl 9-O-dichloroacetyl clavulanate

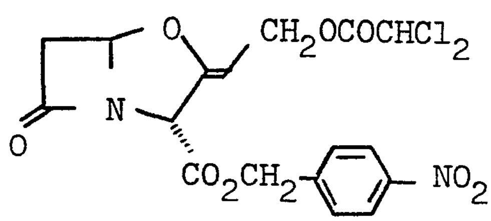

p-Nitrobenzyl clavulanate (20.0g) in dry methylene chloride (200ml) was treated with pyridine (5.1ml) at room temperature. The reaction mixture was cooled to -30°C and dichloroacetyl chloride (5.9ml) in methylene chloride (50ml) was added dropwise. After stirring at -30° for 30 mins the mixture was allowed to warm up to room temperature over 30 mins and was then poured into dilute hydrochloric acid. The organic phase was separated, washed with more dilute hydrochloric acid, water, sodium bicarbonate solution, brine and then dried ($MgSO_4$). After filtration the methylene chloride was removed *in vacuo* to afford a yellow oil which crystallised on trituration with ether. Yield = 24.03g, 90%. A sample re-crystallised from ether gave the following spectral data.

$[\alpha]_D^{20}$ + 30.7° (c. 1%; $CHCl_3$), (Found: C, 46.14; H, 3.08; N, 6.35; Cl, 16.42; $C_{17}H_{14}N_2O_8Cl_2$ requires C, 45.81; H, 3.14; N, 6.29; Cl, 15.92%). $\nu_{max}$ (KBr) 1795, 1745, 1685 and 1605 $cm^{-1}$. $\delta$ ($CDCl_3$) 3.08 (1H, d, *J* 17Hz, 6β-C*H*); 3.55 (1H, dd, *J* 17 and 3 Hz, 6α-C*H*), 4.85 (3H, m, 9-C$H_2$ and 8-C*H*), 5.15 (1H, s, C-3*H*), 5.29 (2H, s, $CO_2CH_2$-$C_6H_4NO_2$), 5.74 (1H, d, *J* 3Hz, C-5*H*), 4.91 (1H, s, C*H*$Cl_2$), 7.50 and 8.24 (each 2H, d, *J* 9Hz, $C_6H_4NO_2$).

0005014

Description 2

N-(2-Furfuryl)N-benzylamine

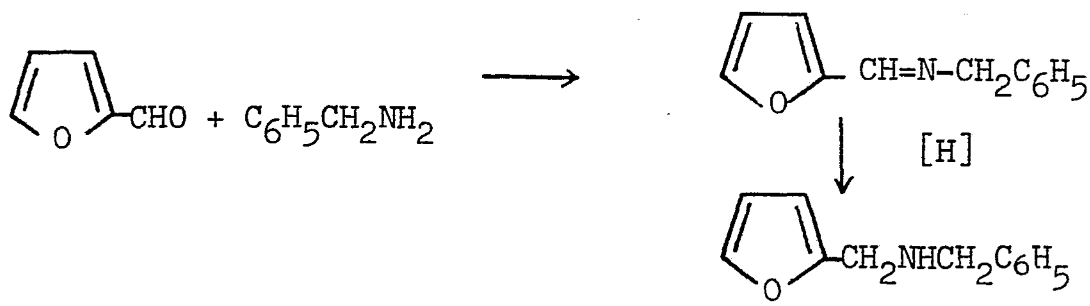


Furfuraldehyde (6.72g)in methanol (80 $cm^3$) at $0^oC$ was treated with benzylamine (7.5 g; 1 equivalent) and stirred for one hour. To the mixture was added water (5 $cm^3$) which was then hydrogenated at atmospheric pressure for 20 minutes in the presence of 1.1 g of palladium on carbon (10%). The mixture was filtered through celite and evaporated in vacuo. The crude product was chromatographed on silica eluting with ethyl acetate/cyclohexane; 1:1. Fractions were collected containing the title compound (detection by aqueous potassium permanganate spray), Rf ($SiO_2$/ethyl acetate/ cyclohexane, 1:1) = 0.6. Combined fractions were evaporated to yield a colourless oil, yield = 7.9g. $\nu$ (film) 3325, 1600, 1495, 1450, 1360, 1335, 1215, 1180, 1145, 1100, 1075, 1030, 1010, 920, 885, 810, 740, 700 $cm^{-1}$. $\delta$ ($CDCl_3$) 1.75 (1H, s, exchanges with $D_2O$, N<u>H</u>), 3.78 (4H, broad, 2 x $NCH_2$), 6.15-6.35 (2H, m, furyl 3- and 4-H), 7.28-7.36 (6H, m, $CH_2C_6H_5$ and furyl 5-H).

EXAMPLE 1

p-Nitrobenzyl 9-N-benzyl-N-(pyrid-2-yl methyl)-aminodeoxyclavulanate.

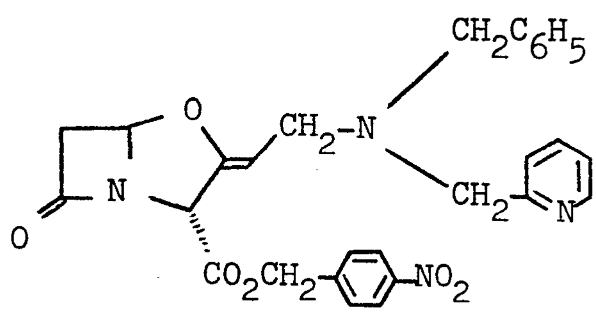

p-Nitrobenzyl dichloroacetylclavulanate (4.51 g) in dry acetonitrile (100ml) at ice temperature was treated with N-benzyl-N-(pyrid-2-yl methyl) amine (4.12 g) in acetonitrile (20ml) for 2 hours. After this time the reaction mixture was poured into brine and extracted with ethyl acetate. The organic phase was washed several times with brine, dried and evaporated *in vacuo* to afford an oil. This was chromatographed on silica gel, eluting with petroleum ether (60-80$^o$) - ethyl acetate (1:1) grading to (1:2). Fractions containing the title compound were combined and evaporated to afford an oil, 0.600 g (11.5%), $\nu_{max}$ (film) 1800, 1752, 1695 $cm^{-1}$; $\delta$ ($CDCl_3$) 2.99 (1H, d, J 17Hz, 6β-CH), 3.21 (2H, s, J 8Hz, 9-$CH_2$), 3.43 (1H, dd, J 17 and 3Hz, 6α-CH), 3.52 and 3.68 (2 x 2H, 2 x s, 2 x N-$CH_2$), 4.79 (1H, t, J 8Hz, 8-CH), 5.07 (1H, s, 3-CH), 5.21 (2H, s, $CO_2CH_2$), 5.60 (1H, d, J 3Hz, 5-CH), 7.0 - 7.75 (10H, m, $C_6H_5$ and

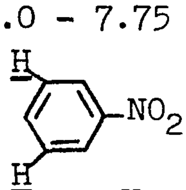 and pyridyl 3-, 4-, 5-H), 8.10 (2H, d, J 9Hz,

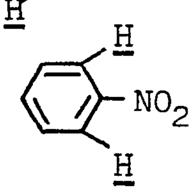 ) and 8.47 (1H, m, pyridyl 6-H).

EXAMPLE 2

Benzyl 9-N-benzyl-N-(pyrid-2-yl methyl)aminodeoxyclavulanate.

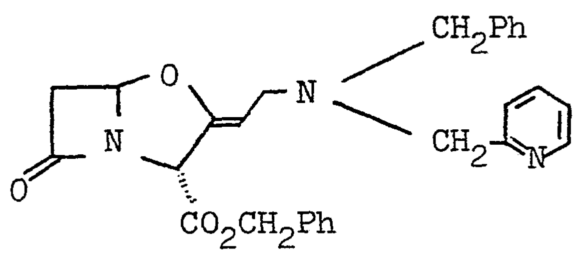

2-Benzylaminomethyl pyridine (6.2g) was dissolved in dry dimethylformamide (60ml) and was added dropwise to an ice-cooled solution of benzyl 9-dichloroacetyl-clavulanate (8.35g) in dry dimethylformamide (80ml). The mixture was then stirred at 4°C for 45 minutes until reaction was complete (t.l.c).

Cold ethyl acetate was then added and the organic mixture was extracted with dilute HCl (3x). The extracts were then combined covered with a cold layer of ethyl acetate and neutralised using solid sodium bicarbonate. Back extraction with ethyl acetate gave a crude product which on chromatography gave the product as a yellow oil 12%; Rf ($SiO_2$; ethyl acetate:pet ether 60:80,1:1)= 0.25, (detection by aqueous potassium permanganate spray).

i.r. $\nu_{max}$ ($CHCl_3$): 1805, 1740, 1725cm$^{-1}$.
n.m.r $\delta$ ($CDCl_3$); 2.95 (1H, d, J 17Hz, 6β-CH), 3.20 (1H, dd, J 17Hz and 3Hz, 6α-CH), 3.52 (2H, s, 9-$CH_2$), 3.70 (2H, s, $CH_2C_5H_4N$), 4.70 (1H, t, J 7Hz, 8-CH), 5.05 (1H, s, 3-CH), 5.15 (4H, s, $CH_2C_6H_5$ x 2), 5.56 (1H, d, J 3Hz, 5α-CH) and 7.40 and 8.48 (m, 14H, 2 x $C_6H_5$ and $C_6H_4N$).

EXAMPLE 3

9-N-Benzyl-N-(pyrid-2-yl methyl) aminodeoxyclavulanic acid.

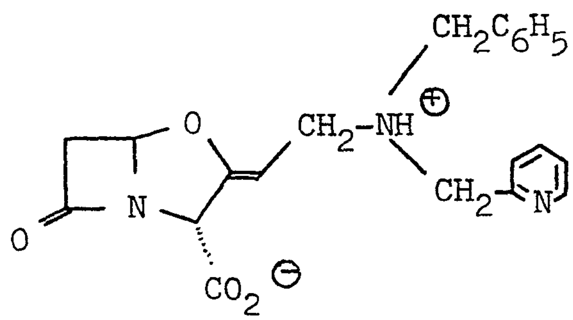

p-Nitrobenzyl 9-N-benzyl-N-(pyrid-2-yl-methyl)-aminodeoxyclavulanate (0.550 g) was hydrogenated at atmospheric pressure in tetrahydrofuran (20ml) and water (2ml) in the presence of 10% Pd-$BaSO_4$ (0.20 g). After 1 hr another portion of catalyst (0.20g) was added and the reduction was continued for a further hour. The catalyst was removed by filtration through celite and the filtrate was evaporated in vacuo. This residue was partitioned between ethyl acetate and water.* The aqueous phase was evaporated to afford a yellow oil (0.17g). This oil was chromatographed on silica gel eluting with chloroform:ethanol 4:1 grading to 1:1. Fractions containing the required compound were combined to afford the tertiary zwitterion as a foam (0.105 g, 26%).

$\nu_{max}$ (film) 3700-2000 (br), 1795, 1695 and 1608 $cm^{-1}$; $\delta$ ($CDCl_3$) 3.09 (1H, d, J 17Hz, 6β-CH), 3.62 (1H, dd, J 17 and 3Hz, 6α-CH), 3.87 (2H, d, J 8Hz, 9-$CH_2$), 4.35 and 4.39 (2 x 2H, 2 x s, $CH_2C_5H_4N$ and $CH_2C_6H_5$), 4.80 (1H, t, J 8Hz, 8-CH), 5.00 (1H, s, 3-CH), 5.78 (1H, d, J 3Hz, 5-CH), 7.28 - 7.61 (7H, m, $C_6H_5$, pyridyl 3- and 5-H), 7.80 - 8.00 (1H, m, pyridyl 4-H) and 8.60 (1H, m, pyridyl 6-H).

* The organic phase from above was evaporated in vacuo to afford recovered starting material (0.260 g). Thus the corrected yield of tertiary zwitterion was 50%.

EXAMPLE 4

9-N-(Pyrid-2-yl-methyl)-aminodeoxyclavulanic acid

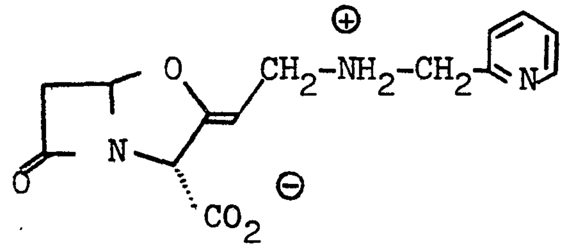

p-Nitrobenzyl 9-N-benzyl-N-(pyrid-2-yl methyl) aminodeoxyclavulanate (0.20 g) in ethyl acetate (2ml) was added to pre-reduced catalyst (0.10 g, 10% Pd/C, 30 mins) in ethanol (10ml) and water (1ml). Reduction was then carried out at 1 atmosphere of hydrogen for 30 min at room temperature. The catalyst was filtered off through celite and the solution was evaporated. The residue was partitioned between ethyl acetate and water. The aqueous phase was evaporated to afford a white crystalline solid (0.075g, 67%) which did not melt but began to decompose above 150°C.

$\nu_{max}$ (nujol) 3500 - 2400 (br), 1803, 1690 and 1610 - 1575 (br) $cm^{-1}$; $\delta$ ($D_2O$) 3.06 (1H, d, *J* 17Hz, 6β-C*H*), 3.57 (1H, dd, *J* 17 and 3Hz, 6α-C*H*), 3.79 (2H, d, *J* 8Hz, 9-C$H_2$), 4.29 (2H, s, C$H_2$), 4.77 (1H, t, *J* 8Hz, 8-C*H*), 5.70 (1H, d, *J* 3Hz, 5-C*H*), 7.28 - 7.55 (2H, m, pyridyl 3- and 5-*H*), 7.65 - 7.95 (1H, m, pyridyl 4-*H*) and 8.40 - 8.60 (1H, m, pyridyl 6-*H*).

EXAMPLE 5

p-Nitrobenzyl 9-N-benzyl-N-(N'-methylpyrrole-2-methyl)-aminodeoxyclavulanate.

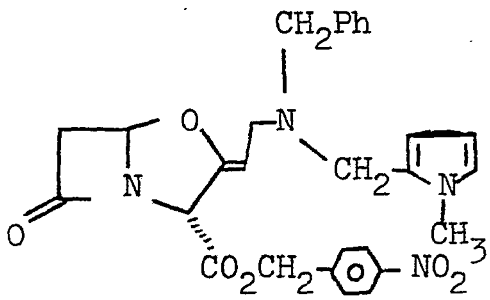

1-Methyl-2-benzylaminomethyl-pyrrole (4g) was dissolved in acetonitrile (40ml) and was added dropwise to a solution of p-nitrobenzyl 9-dichloroacetylclavulanate (4.68g) in acetonitrile (45ml). The reaction was stirred initially at -20°C and then allowed to warm to room temperature over a period of 5 hours.

The acetonitrile was then removed under reduced pressure and ethyl acetate (100ml) was then added. The organic solution was then washed with water (3 x) and brine. The organic solution was then dried ($MgSO_4$) and evaporated to an oil. Silica gel chromatography afforded the required product as a gum. (19%) Rf ($SiO_2$; ethyl acetate : pet. ether 60-80°; 1:1)=0.60 (detection by aqueous potassium permanganate).

$\nu_{max}$ ($CHCl_3$): 1800, 1745 and 1695cm$^{-1}$. δ ($CDCl_3$): 2.85 (1H, d, J 17Hz, 6β-CH); 3.10 (1H, dd, J 17 and 3 Hz, 6α-CH); 3.39 (9H, m, N-$CH_3$, $CH_2$-pyrrole, $CH_2C_6H_4NO_2$, 9-$CH_2$); 4.73 (1H, broad t, J 7Hz, 8-CH); 5.10 (1H, s, 3-CH); 5.20 (2H, s, $CH_2C_6H_5$); 5.58 (1H, d, J 3Hz, 5-CH); 5.95 (2H, d, J 4Hz, pyrrole 3- and 4-H), 6.48 (1H, t, J 2Hz, pyrrole 5-H), 7.21 (5H, s, $CH_2C_6H_5$), 7.39 and 8.08 (both

2H, d, _J_ 9Hz; $C_6\underline{H}_4NO_2$).

EXAMPLE 6

9-N-Benzyl-N-(N'-methylpyrrole-2-methyl) aminodeoxyclavulanic acid.

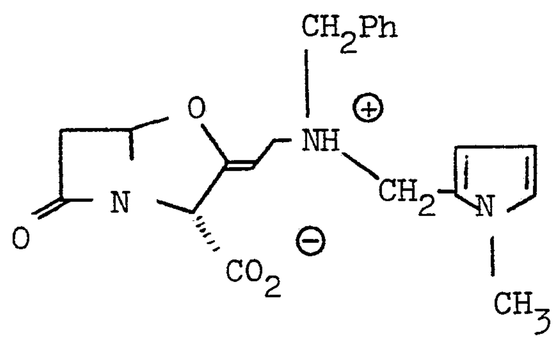

p-Nitrobenzyl 9-N-benzyl-N-(N'-methylpyrrole-2-methyl) aminodeoxyclavulanate (0.200 g) was hydrogenated at atmospheric pressure in tetrahydrofuran (10ml) and water (1ml) in the presence of 10% Pd-$BaSO_4$ (0.066 g) for 30 minutes. The catalyst was removed by filtration through celite and the filtrate was evaporated under reduced pressure. This residue was partitioned between ethyl acetate and water. The aqueous phase was evaporated to afford a yellow oil (0.12 g). This oil was chromatographed on silica gel, eluting with chloroform: ethanol 3:1 grading 1:1. Fractions containing the required compound were combined to afford the tertiary zwitterion as a foam (0.065g; 45%). Rf ($SiO_2$; chloroform:ethanol 1:1) 0.50 (detection by Erlichs reagent).

$\nu_{max.}$ ($CHCl_3$): 1790, 1695 and 1610 $cm^{-1}$.

n.m.r. spectrum ($D_2O$) includes the following peaks at, $\delta$: 3.04 (1H, d, J 17Hz, 6β-CH), 3.37 (s, 3H, N-$CH_3$), 5.69 (1H, d, J 3Hz, C-5H), 6.10 (1H, m, pyrrole-CH), 6.24 (1H, m, pyrrole-CH), 6.73 (1H, m, pyrrole-CH), 7.37 (5H, s, $CH_2C_6H_5$).

## EXAMPLE 7

**p-Nitrobenzyl 9-N-benzyl-N-(thienyl-2-methyl)aminodeoxy-clavulanate**

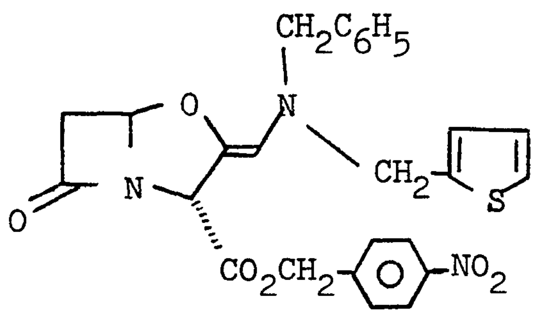

p-Nitrobenzyl 9-O-dichloroacetyl-clavulanate (5.19g) in dry acetonitrile (60ml) was treated at $0^{o}C$ by dropwise addition of 2-N-benzylaminomethylthiophene (4.50g) in dry acetonitrile (30ml). After stirring at $0^{o}C$ for 3 hours the acetonitrile was evaporated off under reduced pressure. The residue was then dissolved in ethyl acetate (100ml). This organic solution was then washed with water and then dried ($MgSO_4$). After filtration the ethyl acetate was removed *in vacuo* to afford a yellow oil which on column chromatography gave the title compound as an oil. Yield 1.20 g; 20%.

$\nu_{max}$ ($CHCl_3$):1805, 1750, 1700 and 1605 $cm^{-1}$. $\delta$ ($CDCl_3$) 2.95 (1H, d, *J* 17Hz, 6β-C*H*), 3.18 (2H, d, *J* 7Hz, 9-C$H_2$), 3.40 (1H, dd, *J* 17 and 3 Hz, 6α-C*H*), 3.54 (2H, s, C$H_2$-thiophene),3.67 (2H, s, N-C$H_2$Ph), 4.73 (1H, broad t, *J* 7Hz, 8-C*H*), 5.08 (1H, m, 3-C*H*), 5.20 (2H, s, $CO_2CH_2$-), 5.60 (1H, d, *J* 3Hz, 5-C*H*), 6.92 and 7.18 (3H, m, thienyl-*H*), 7.26 (5H, s, N-$CH_2C_6H_5$), 7.42 and 8.10 (both 2H, d, *J* 9Hz, $C_6H_4NO_2$).

EXAMPLE 8

Benzyl 9-[N-(2'-furfuryl)-N-benzylamino]deoxy-clavulanate

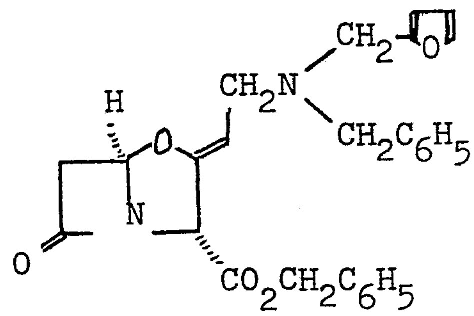

Benzyl dichloroacetylclavulanate (8.32g) in dry dimethylformamide (50 $cm^3$) at $0^o$ was treated with N-(2'-furfuryl)-N-benzyl-amine (7.4g; 1.9 equivalents) and stirred at $0^o$ for 2½ hours, then poured into cold ethyl acetate (150 $cm^3$) and washed with water (5 x 50 $cm^3$) and saturated brine (5 x 50 $cm^3$), dried (anhydrous magnesium sulphate) and evaporated in vacuo to yield a coloured oil, yield = 11g. 1.2g of this crude product was chromatographed on silica eluting with ethyl acetate/cyclohexane; 1:1. Fractions were collected containing the title compound; Rf ($SiO_2$/ ethyl acetate: cyclohexane; 1:1)= 0.78 (detection by aqueous potassium permanganate spray). Combined fractions were evaporated in vacuo to yield an oil, 0.31g. $\nu$ (film) 1805, 1750, 1692, 1495, 1450, 1305, 1227, 1175, 1150, 1120, 1075, 1045, 1015, 965, 755, 700 $cm^{-1}$. $\delta$ ($CDCl_3$) 2.91 (1H, d, J 17Hz, 6β-CH), 3.15 (2H, d, J 7Hz, 9$CH_2$), 3.37 (1H, dd, J 17 and 3Hz, 6α-CH), 3.47 (4H broad s, 2 x $NCH_2$), 4.70 (1H, dt, J 7 and 1Hz, 8-CH), 5.00 (1H, d, J 1Hz, 3-CH), 5.11 (2H, S, $OCH_2C_6H_5$), 5.55 (1H, d, J 3Hz, 5-CH), 6.05-6.25 (2H, m, furyl 3- and 4-H), 7.21 (11H, broad s, 2 x $C_6H_5$ and furyl 5-H).

EXAMPLE 9

9-[N-(2'-Furfuryl)-N-benzylamino]deoxyclavulanic acid

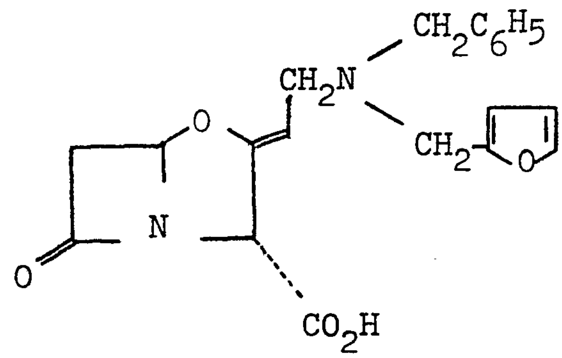

Benzyl 9-[N-(2'-furfuryl)-N-benzylaminodeoxy]clavulanate (2.8g) in tetrahydrofuran (100 $cm^3$) and water (5 $cm^3$) was hydrogenated at atmospheric pressure in the presence of 0.9g palladium on charcoal (10%). The mixture was filtered through celite and the catalyst washed with aqueous tetrahydrofuran (50 $cm^3$). The filtrate was evaporated *in vacuo* to yield a foam. This foam was chromatographed on cellulose eluting with butanol/propan-2-ol/water; 7:7:1. Fractions were collected containing material Rf ($SiO_2$/butanol/propan-2-ol/water; 7:7:6)=0.67 (detection by aqueous potassium permanganate spray). Combined fractions were evaporated in vacuo to yield an oil which on trituration with diethyl ether yielded the title compound as colourless crystals; yield=42mg. $\nu$ (Nujol) 1780, 1690, 1650, 1305, 1145, 1140, 1120, 1035, 1015, 935, 925, 895, 750, 740, 705, 655 $cm^{-1}$. $\delta$ ($D_2O/CD_3OD$) 2.97 (1H, d, *J* 17Hz, 6β-C*H*), 3.54 (1H, dd, *J* 17 and 3Hz, 6α-C*H*), 3.59 (2H, d, *J* 8Hz, 9C$\underline{H}_2$), 4.07 (2H, AA'q, *J* 14Hz, C$\underline{H}_2$-furyl), 4.12 (2H, s, C$\underline{H}_2C_6H_5$), 4.8 (HOD obscuring 8-C*H*), 4.91 (1H, S, 3C*H*), 5.75 (1H, d, *J* 3Hz, 5α-C*H*), 6.43 - 6.58 (2H, m, furyl 3- and 4-*H*), 7.42 (5H, s, $C_6\underline{H}_5$), 7.59 (1H, m, furyl 5-*H*).

0005014

EXAMPLE 10

9-[N-(2'-Furfuryl)]aminodeoxyclavulanic acid

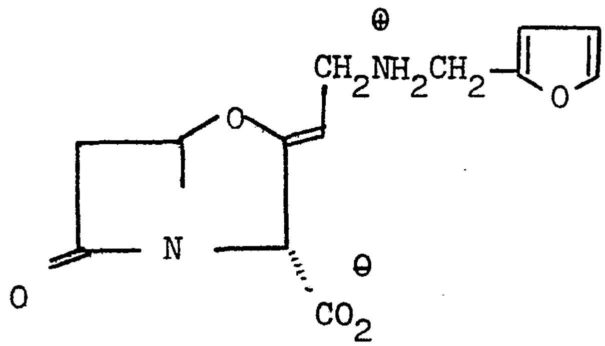

Benzyl 9-[N-(2'-furfuryl)-N-benzylamino]deoxyclavulanate (2.8g) in tetrahydrofuran (100 $cm^3$) and water (5 $cm^3$) was hydrogenated at atmospheric pressure for 10 minutes in the presence of 0.9g palladium on charcoal (10%). The mixture was filtered through celite and the catalyst washed with aqueous tetrahydrofuran (50 $cm^3$). The filtrate was evaporated in vacuo to yield a foam. This foam was chromatographed on cellulose eluting with butanol/propan-2-ol/water 4:4:1. Fractions were collected containing material Rf0.53($SiO_2$/butanol-propan-2-ol-water; 7:7:6), detection by aqueous potassium permanganate spray. Combined fractions were evaporated in vacuo to yield an oil which on trituration with n-butanol gave colourless crystals, 5 mg. $\nu$ (Nujol) 3570,3350,2700,2400(all broad) 1805,1690, 1610, 1300, 1185, 1155, 1140, 1120, 1080, 1045, 1015, 945, 930, 895, 885, 745 $cm^{-1}$. $\delta$ ($D_2O$) 3.06 (1H, d, J 17Hz, 6β-C<u>H</u>), 3.55 (1H, dd, J 17 and 3Hz, 6α-C<u>H</u>), 3.67 (2H, d, J 8Hz, 9C$\underline{H}_2$), 4.18 (2H, s, C$\underline{H}_2$-furyl), 4.75-5.05 (2H, m, 3C<u>H</u> and 8-C<u>H</u>), 5.71 (1H, d, J 3Hz, 5-C<u>H</u>), 6.37 - 6.55 (2H, m, furyl 3- and 4-<u>H</u>), 7.52 (1H, m, furyl 5-<u>H</u>).

EXAMPLE 11

Benzyl 9-N-(pyridyl-4-methyl)benzylaminodeoxyclavulanate

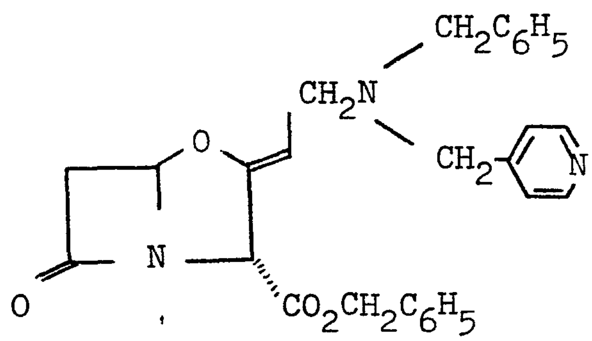

Benzyl dichloroacetylclavulanate (5.5g) in dry dimethylformamide (50 $cm^3$) at $0^o$ was treated with N-(pyridyl-3-methyl)-N-benzylamine (1.9 equivalents) dropwise in dimethylformamide ($40cm^3$) and stirred for 4 hours at $0^o$; then poured into ethylacetate ($150cm^3$) and washed with water (5 x $50cm^3$) and saturated brine (3 x $50cm^3$), dried (anhydrous magnesium sulphate) and evaporated in vacuo to yield a coloured oil. This oil was chromatographed on silica gel, eluting with ethyl acetate-cyclohexane (1:1) grading to (3:1). Fractions were collected containing the title compound, Rf ($SiO_2$/ethylacetate - cyclohexane; 1:1) = 0.3. The combined fractions were evaporated in vacuo to yield an oil, yield = 0.67g (10%). $\nu$ (film) 1805, 1750, 1690, 1600, 1495, 1450, 1415, 1305, 1230, 1180, 1120, 1045, 1020, 890, 800, 750, 700 $cm^{-1}$. $\delta$($CDCl_3$) 2.91 (1H, d, J 17Hz, 6β-CH), 3.13 (2H, d, J 7Hz, $9CH_2$), 3.41 (1H, dd, J 17 and 3 Hz, 6α-CH), 3.44, 3.47 (2 x 2H, 2 x s, 2 x $NCH_2$), 4.72 (1H, t, J 7Hz, 8CH), 5.05 (1H, s, 3CH), 5.15 (2H, s, $CO_2CH_2C_6H_5$), 5.57 (1H, d, J 3Hz, 5αCH), 7.18 - 7.35 (12H, m, 2 x $CH_2C_6H_5$, pyridyl 3- and 5-H), 8.44 - 8.5 (2H, m, pyridyl 2- and 6-H).

EXAMPLE 12

Benzyl 9-N-(pyridyl-3-methyl)benzylaminodeoxyclavulanate

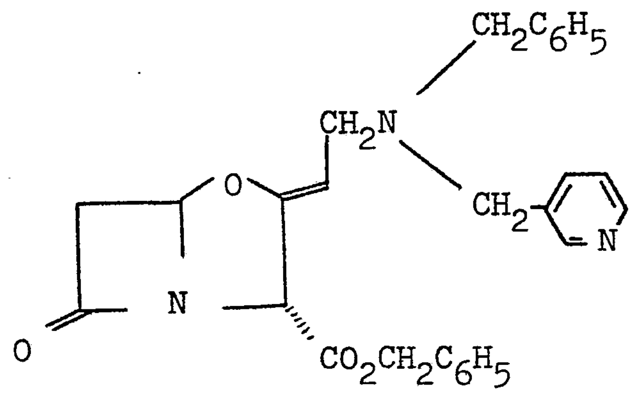

Benzyl dichloroacetyl clavulanate (6 g) in dry dimethylformamide ($70cm^3$) at $0^o$ was treated with N-(pyridyl-3-methyl)-N-benzylamine (1.9 equivalents), dropwise in dimethylformamide ($30cm^3$) and stirred for 5 hours; then poured into ethyl acetate ($250cm^3$) and washed with water (4 x $100cm^3$) and brine (4 x $100cm^3$), dried (anhydrous magnesium sulphate) and evaporated in vacuo to an oil. This oil was chromatographed on silica eluting with ethyl acetate/cyclohexane, 1:1. Fractions were collected containing the title compound Rf ($SiO_2$/ethylacetate-cyclohexane, 1:1) = 0.34 (detection by potassium permanganate spray). The combined fractions were evaporated in vacuo to yield an oil, 0.57g (8%). $\nu$(film) 1800, 1745, 1690, 1450, 1420, 1300, 1180, 1120, 1040, 1015, 790, 745, 715, 700 $cm^{-1}$. $\delta$ ($CDCl_3$) 2.94 (1H, d, J 17Hz, 6β-CH), 3.11 (2H, d, J 7Hz, $9CH_2$), 3.41 (1H, dd, J 17 and 3Hz, 6αCH), 3.43, 3.46 (4H, 2 x s, 2 x $NCH_2$), 4.71 (1H, t, J 7Hz, 8CH), 5.04 (1H, s, 3CH), 5.15 (2H, s, $CO_2CH_2C_6H_5$), 5.58 (1H, d, J 3Hz, 5αCH), 7.10-7.66 (2H, m, pyridyl 4- and 5-H), 7.27 (10H, broad s, 2 x $CH_2C_6H_5$) 8.48 (2H, m, pyridyl 2- and 6-H).

EXAMPLE 13

9-(3'-Pyridyl methyl)aminodeoxyclavulanic acid

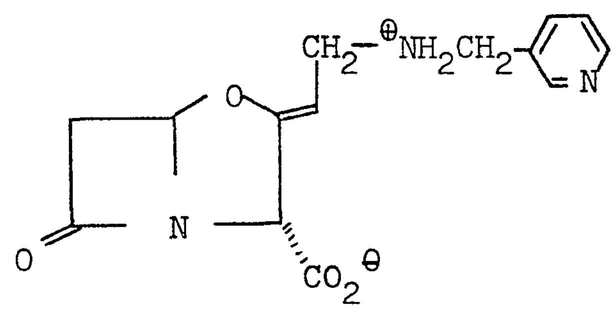

Benzyl 9-N-(pyridyl-3'-methyl) benzylaminodeoxyclavulanate (0.46g) in tetrahydrofuran ($40cm^3$) and water ($3\ cm^3$) was hydrogenated at atmospheric pressure in the presence of 10% palladium on carbon (0.15g) for 2½ hours. The mixture was filtered through a fibre glass filter and the catalyst washed with aqueous tetrahydrofuran ($50cm^3$). The filtrate was evaporated *in vacuo* to an oil. This crude product was chromatographed on cellulose, eluting with butanol-propan-2-ol-water; 4:4:1 graduating to 4:4:2. Fractions were collected containing the title compound Rf ($SiO_2$/butanol-propan-2-ol-water; 7:7:6) = 0.29 (detection by potassium permanganate spray).

Combined fractions were evaporated *in vacuo*, addition of cold ethanol yielded colourless crystals, 22 mg (8%) $\nu$(Nujol) (3700-3100), (2800-2500), (2500-2300), 1810, 1690, 1610, 1580, 1400, 1305, 1185, 1125, 1085, 1070, 1050, 1020, 990, 950, 900, 810, 715 $cm^{-1}$. $\delta$ ($D_2O/CD_3OD$ 10%) 3.09 (1H, d, J 17Hz, 6βCH) 3.59 (1H, dd, J 17Hz and 3Hz, 6αCH), 3.77 (2H, d, J 7Hz, $9CH_2$), 4.25 (2H, s, $NCH_2$), 4.32 (1H, t, J 7Hz, 8CH), 4.98 (1H, s, 3CH), 5.74 (1H, d, J 3Hz, 5αCH). 7.41 - 7.58 (1H, m, pyridyl 5-H), 7.87 - 7.97 (1H, m, pyridyl 4-H), 8.55 (2H, broad m, pyridyl 2- and 6-H).

EXAMPLE 14

Benzyl 9-N,N-di(pyrid-3'-ylmethyl)aminodeoxyclavulanate

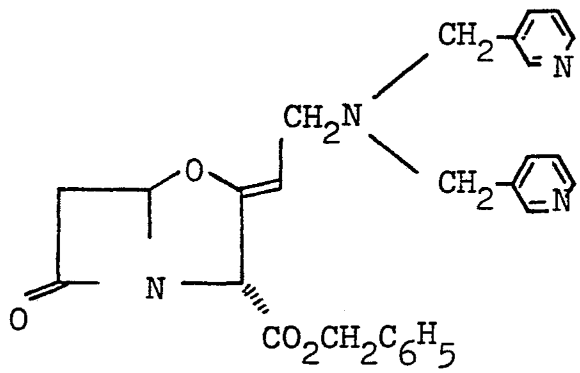

Benzyl dichloroacetylclavulanate (6g) in dry dimethylformamide ($50cm^3$) at $0^o$ was treated with N, N-di(pyrid-3-ylmethyl)-amine (1.9 equivalents) and stirred at $0^o$ for three hours then at $20^o$ for 3 hours. The mixture was poured into ethyl acetate ($250cm^3$) and washed with water (5 x $50cm^3$) and saturated brine (5 x $50cm^3$), dried (anhydrous magnesium sulphate) and evaporated in vacuo to yield an oil. This was chromatographed on silica eluting with ethyl acetate - chloroform - ethanol; 9:3:1. Fractions were collected containing the title compound, Rf ($SiO_2$/ethyl acetate - chloroform - ethanol, 9:3:1) = 0.24 (detection by aqueous potassium permanganate spray). Combined fractions were evaporated in vacuo to yield the title compound as an oil, 0.79g. $\nu$(film) 1800, 1745, 1690, 795, 745, 715, 700 $cm^{-1}$. $\delta$ ($CDCl_3$) 2.98 (1H, d, J 17Hz, 6β-CH), 3.12 (2H, d, J 7Hz, $9CH_2$), 3.43 (1H, dd, J 17 and 3Hz, 6αCH), 3.45 (4H, s, 2 x $NCH_2$), 4.69 (1H, t, J 7Hz, 8CH), 5.05 (1H, s, 3CH), 5.18 (2H, s, $CO_2CH_2$), 5.61 (1H, d, J 3Hz, 5αCH), 7.13 - 7.65 (4H, m, 2 x pyridyl 4- and 5-CH), 7.28 (5H, s, $CH_2C_6H_5$), 8.47 (4H, broad m, 2 x pyridyl 1- and 6-CH).

EXAMPLE 15

9-N-(Pyrid-3-ylmethyl)aminodeoxyclavulanic acid

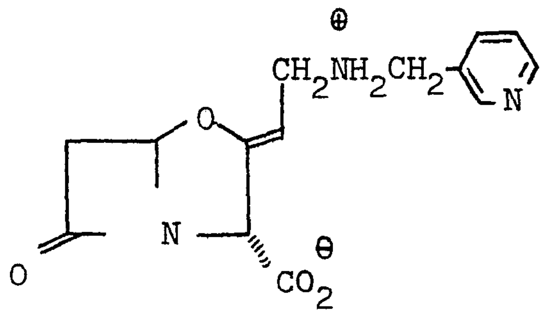

Benzyl 9-N,N-di(pyrid-3'-ylmethyl)aminodeoxyclavulanate (0.68g) in tetrahydrofuran ($35cm^3$) and water ($4cm^3$) was hydrogenated in the presence of palladium on carbon (30% palladium), (140 mg) for five hours at atmospheric pressure. A further 100 mg of the same catalyst was added and hydrogenolysis continued for a further 1½ hours. The catalyst was filtered off and washed with aqueous ethanol ($100 cm^3$), the filtrate was evaporated in vacuo to yield a colourless crystalline solid. This was washed with cold ethanol and dried, yield = 80 mg. The washings and supernatant were evaporated in vacuo to yield an oil. This oil was dissolved in tetrahydrofuran ($2.0cm^3$) and water ($10cm^3$) and hydrogenolysed in the presence of palladium on carbon (30% palladium; 150 mg) at atmospheric pressure for 21 hours. The catalyst was filtered off and washed with aqueous ethanol ($50cm^3$) and the filtrate evaporated in vacuo to yield a solid; cold ethanol was added and the solid filtered off. After washing with cold ethanol, the solid was dried to yield a further 82 mg of the title compound. Thin layer chromatography and infrared spectroscopy showed both batches to be identical. Total yield = 162 mg Rf ($SiO_2$/ butanol - propan-2-ol-water; 7:7:6) = 0.24. ν (Nujol) (3700-3100), (2800-2500), (2500-2300), 1810, 1690, 1610, 1580, 990, 950, 900, 810, 715 $cm^{-1}$.

EXAMPLE 16

Benzyl 9-N-(2-methylallyl)-N-(1-methylpyrrole-2-methyl)-aminodeoxyclavulanate.

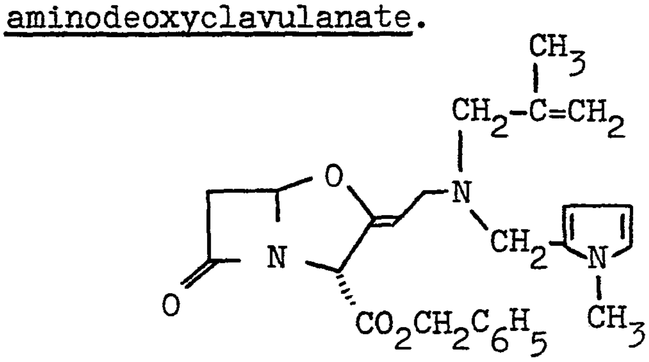

Benzyl 9-O-dichloroacetylclavulanate (5.58g) in acetonitrile (60 ml) was treated at $0^oC$ with dropwise addition of 1-methyl-2-(2-methylallylaminomethyl)-pyrrole (4.35g) in acetonitrile (40ml). After stirring at $0^oC$ for two hours the acetonitrile was removed under reduced pressure. The resulting oil was then dissolved in ethyl acetate (100ml) and washed with water, dried ($MgSO_4$) and evaporated in vacuo. Column chromatography of the crude product afforded the title compound as a gum. Yield 1.10g; 18%. $\nu_{max}$ ($CHCl_3$) 1800, 1745, 1690 and 1640 $cm^{-1}$. $\delta$ ($CDCl_3$) 1.64 (3H, s, $CH_2$-C($C\underline{H}_3$)=), 2.80 (2H, s, $C\underline{H}_2$-C($CH_3$)=), 2.95 (1H, d, J 17Hz, 6β-C$\underline{H}$), 3.06 (2H, d, J 7Hz, 9-$C\underline{H}_2$), 3.35 (2H, s, $C\underline{H}_2$-pyrrole), 3.40 (1H, dd, J 17 and 3Hz, 6α-C$\underline{H}$), 3.53 (3H, s, N-$C\underline{H}_3$), 4.68 (1H, broad t, J 8-C$\underline{H}$), 4.80 (2H, s, C=$C\underline{H}_2$), 5.17 (2H, s, $CO_2C\underline{H}_2$), 5.25 (1H, s, 3-C$\underline{H}$), 5.60 (1H, d, J 3Hz, 5-C$\underline{H}$), 5.95 and 6.52 (3H, m, pyrrole-$\underline{H}$) and 7.30 (5H, s, $C_6\underline{H}_5$).

EXAMPLE 17

9-N-(1-Methylpyrrole-2-methyl)-aminodeoxyclavulanic acid.

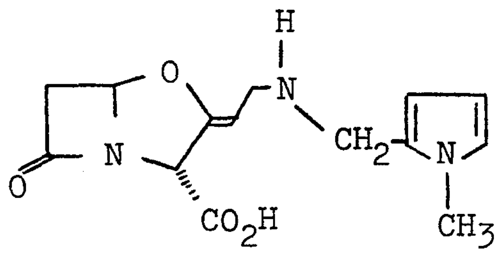

Benzyl 9-N-(2-methylallyl)-N-(1-methylpyrrole-2-methyl)-aminodeoxyclavulanate (1.10g) in ethanol (20ml) was carefully added to a pre-hydrogenated mixture of 10% palladium on charcoal (550mg) in ethanol (20ml). The mixture was then hydrogenated at atmospheric pressure for 30 minutes. The catalyst was then filtered through celite and the filter-cake washed well with ethanol. The ethanol was then evaporated under reduced pressure and the residue crystallised on trituration with ethanol to afford the required compound as a white crystalline solid. Yield 39%.

$\nu_{max}$ (nujol): 1795, 1690, 1643 and 1600 $cm^{-1}$.

$\delta$ ($D_2O$): 3.05 (1H, d, J 17Hz, 6β-CH); 3.56 (3H, s, $CH_3$), 3.60 (1H, dd, J 17 and 3Hz, 6α-CH); 3.67 (2H, d, J 7Hz, 9-$CH_2$); 4.14 (2H, s, $CH_2$-pyrrole); 4.76 (1H, broad t, J 7Hz, 8-CH); 4.95 (1H, s, 3-CH); 5.71 (1H, d, J 3Hz, 5-CH), 6.10 (1H, t, J 3Hz, pyrrole-CH), 6.25 (1H, m, pyrrole-CH); and 6.75 (1H, t, J 3Hz, pyrrole-CH).

EXAMPLE 18

Benzyl 9-N-benzyl-N-(quinol-3-yl)aminodeoxyclavulanate

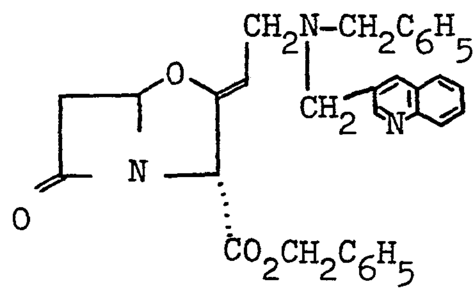

To a solution of benzyl dichloroacetylclavulanate (4g), in dimethylformamide (100ml) at ice temperature was added, dropwise, 3-benzylaminomethylquinoline (4.7g), in dimethylformamide (15ml). After 4 hours at $0^oC$ the reaction mixture was poured into brine and extracted with ethyl acetate. The organic phase was washed several times with brine, then dried ($MgSO_4$) and evaporated to a dark yellow oil. Chromatography on silica gel (elution with petrol: ethyl acetate 4:1 grading to 1:1) afforded the title compound as an oil, 0.89g (17%).

I.r. (film) 1802, 1750 and 1695 $cm^{-1}$.

N.m.r. $\delta$($CDCl_3$) 2.91 (1H, d, J 17Hz, 6β-CH), 3.18 (2H, d, J 8Hz, 9-$CH_2$), 3.38 (1H, dd, J 17 and 3Hz, 6α-CH), 3.52 and 3.61 (both 2H, s, $CH_2C_6H_5$ and $CH_2C_9NH_6$), 4.77 (1H, t, J 8Hz, 8-CH), 5.06 (1H, s, 3-CH), 5.16 (2H, s, $CO_2CH_2$ ), 5.58 (1H, d, J 3Hz, 5-CH), 7.27 (5H, s, $C_6H_5$), 7.46 - 8.13 (5H, m, quinolyl 4-, 5-, 6-, 7- and 8-H), and 8.85 (1H, m, quinolyl 2-H).

Example 19

Benzyl 9-N-(2′-methylallyl)-N-(thien-2″-ylmethyl) aminodeoxyclavulanate

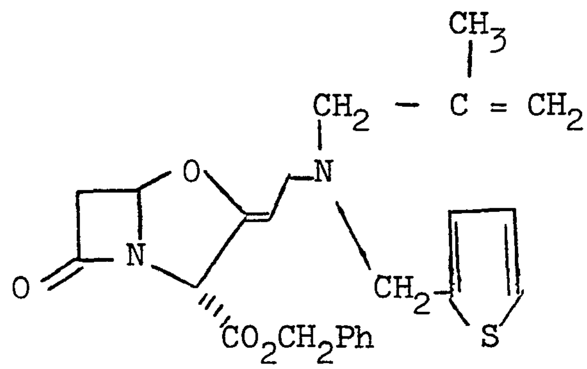

Benzyl 9-O-dichloroacetylclavulanate (8.57 g) in acetonitrile, at $0^oC$ was treated with dropwise addition of N-(2-methylallyl)-N-(thien-2′-ylmethyl)amine (6.8 g) in acetonitrile. On final addition the reaction was stirred at $0^oC$ for 1 hour and then for a further 2 hours at between 0 and $20^oC$.

The acetonitrile was then removed under a reduced pressure and the resulting oil was dissolved in ethylacetate. The solution was then washed with water, dried ($MgSO_4$) and evaporated *in vacuo*. Silica-gel chromatography afforded the title compound as a colourless oil in 10% yield.

$\nu$ max ($CHCl_3$): 1800, 1745, 1695, and 1610cm$^{-1}$.

$\delta$($CDCl_3$): 1.74 (3H, s, $CH_3$); 2.98 (2H, s, N-$CH_2$-C=$CH_2$); 2.99 (1H, d, J 17 Hz, 6β-CH); 3.14 (2H, d, J 7 Hz, 9-$CH_2$); 3.40 (1H, dd, J 17 and 3 Hz; 6α-CH); 3.62 (2H, s, $CH_2$ -thienyl); 4.70 (1H, broad t, J 7 Hz, 8-CH); 4.84 (2H, broad s, C=$CH_2$); 5.05 (1H, s, C-3H); 5.12 (2H, centre of AA′ system $CO_2CH_2$); 5.59 (1H, d, J 3 Hz, C-5H); 7.00 (3H, m, thienyl-H), and 7.30 (5H, s, $CH_2C_6H_5$).

Example 20

Benzyl 9-N-(indol-3′-ylmethyl)-N-(2″-methylallyl) aminodeoxyclavulanate

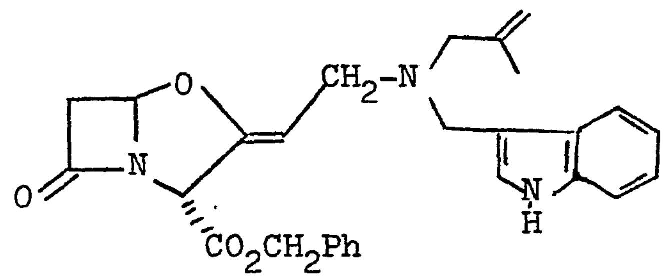

N-(Indol-3-ylmethyl)-N-(2′-methylallyl)amine (3.9 g) in dimethylformamide (10 ml) was added dropwise to a stirred solution of benzyl dichloroacetylclavulanate (4.1 g) in dimethylformamide (50 ml) at 0°C. After 50 minutes at this temperature the reaction mixture was poured into brine and extracted with ethyl acetate. The organic phase was washed with brine ( 4x ), dried ($MgSO_4$) and evaporated to a brown oil which was chromatographed on silica gel (elution; petrol:ethylacetate 5:2 grading to 2:1) to give the title compound as an oil (1.65 g; 34%).

$\nu$ max ($CHCl_3$) 3500, 1802, 1750, and 1700 $cm^{-1}$.

N.M.R. $\delta$ ($CDCl_3$) 1.71 (3H, s, $CH_3$), 2.88 (1H, d, J 17 Hz, 6β-CH), 2.91 (2H, s, $CH_2$-C=C), 3.14 (2H, d, J, 7 Hz, 9-$CH_2$), 3.38 (1H, dd, J 17 and 3 Hz, 6α-CH), 3.71 (2H, s, -$CH_2$-het), 4.78 (1H, bt, 8-CH), 4.85 (2H, bs, =$CH_2$), 5.03 (1H, s, 3-CH), 5.15 (2H, s, $CO_2CH_2Ph$), 5.54 (1H, d, J 3 Hz, 5-CH), 6.85 - 7.80 (5H, m, indolyl-H), 7.27 (5H, s, $C_6H_5$), and 8.08 (1H, bs, NH).

Example 21

9-N-(Indol-3'-ylmethyl)aminodeoxyclavulanic acid

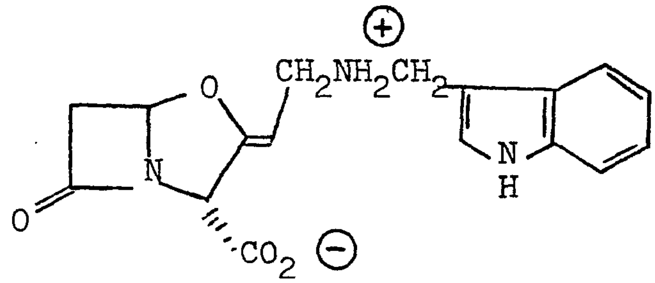

10% Palladium on carbon (0.120 g) in ethanol (20 ml) was hydrogenated at atmospheric pressure for 15 minutes. After this time benzyl 9-N-(indol-3'-ylmethyl)-N-(2''-methylallyl)aminodeoxyclavulanate (1.72 g) in ethanol (5 ml ) was added and the hydrogenation was continued for 30 minutes. The catalyst was filtered off and the solvent removed in vacuo to afford a yellow oil which crystallised from methanol. Yield of the title compound was 0.60 g, 50%.

νmax (KBr). 1783 (br), 1685, 1620 (br), 1308 and 740 $cm^{-1}$.

N.M.R. $D_2O$-DMSO-$d_6$, 3.02 (1H, d, J 17 Hz, 6β-CH), 3.58 (1H, dd, J 17 and 3 Hz, 6α-CH), 3.73 (2H, d, J 8 Hz, 9-$CH_2$), 4.26 (2H, s, $NH_2$-$CH_2$), 4.93 (1H, s, 3-CH), 5.73 (1H, d, J 3 Hz, 5-CH), 7.15 - 7.80 (5H, m, indolyl-2,4,5,6 and 7-H).

8-CH obscured by HOD.

Example 22

Benzyl 9-N-(2′-methylallyl)-N-(quinol-3″-ylmethyl) aminodeoxyclavulanate

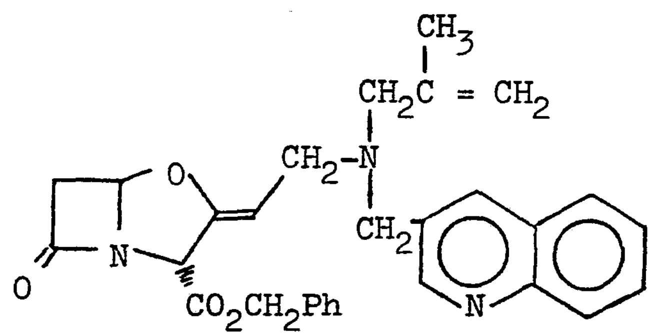

N-(2-methylallyl)-N-(quinol-3′-ylmethyl)amine (3.4 g) in dimethyl-formamide (20 ml) was added dropwise to a stirred solution of benzyl dichloroacetylclavulanate (3.4 g) in dimethylformamide (70 mls) at ice temperature. After 3 hr. at this temperature the reaction mixture was poured into brine and extracted with ethyl acetate. The organic phase was washed with brine (3x) and then extracted with dilute hydrchloric acid. The acidic phase was neutralised with solid sodium bicarbonate and extracted with fresh ethyl acetate. The latter phase was washed with brine, dried ($MgSO_4$) and evaporated to a red-brown oil. Chromatography on silica gel (elution : petrol/ethyl acetate 5/2 - 1/1) afforded the title compound as an oil (0.270 g; 4%).

$\nu$max (film), 2900, 1800, 1745, 1695, 900 and 760 $cm^{-1}$.

<u>N.M.R.</u> δ($CDCl_3$) 1.73 (3H, s, $C\underline{H}_3$) , 2.91 (2H, s, $C\underline{H}_2$), 2.93 (1H, d, <u>J</u> 17 Hz, 6β-C<u>H</u>), 3.14 (2H, d, <u>J</u> 8 Hz, 9-$C\underline{H}_2$), 3.41 (1H, dd, <u>J</u> 17 and 3 Hz, 6α-C<u>H</u>), 3.59 (2H, s, quinoline-$C\underline{H}_2$), 4.75 (1H, bt, <u>J</u> 8 Hz, 8-C<u>H</u>), 4.89 (2H, bs, = $CH_2$), 5.09 (1H, s, 3-C<u>H</u>), 5.18 (2H, s, $C\underline{H}_2Ph$), 5.60 (1H, d, <u>J</u> 3 Hz, 5-C<u>H</u>), 7.20 - 8.20 (10H, m, $C_6\underline{H}_5$ and quinoline 4-<u>H</u>, 5-<u>H</u>, 6-<u>H</u>, 7-<u>H</u> and 8-<u>H</u>) and 8.85 (1H, m, quinoline 2-<u>H</u>).

Example 23

Benzyl 9-N-methyl-N-(pyrid-3-ylmethyl)aminodeoxyclavulanate

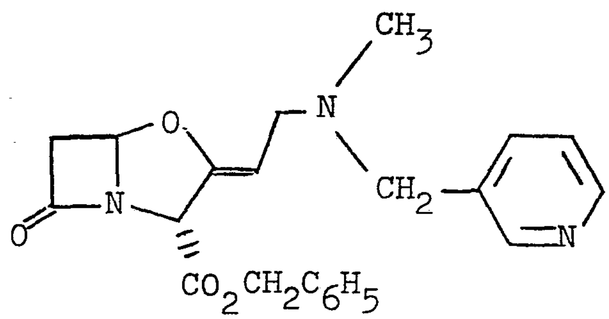

Benzyl dichloroacetylclavulanate (11.2 g) in dimethylformamide (50 ml) was treated with a solution of N-methyl-N-(pyrid-3-ylmethyl)amine (6.5 g) in dimethylformamide (5 ml), at $0^{o}$. The stirred reaction mixture was maintained at $0^{o}$ for 5 hours and poured into ethyl acetate (250 ml); washed with water (2 x 100 ml) and brine (2 x 100 ml), dried (magnesium sulphate) and evaporated to an oil. Chromatography on silica gel, eluting with methyl acetate : ethanol, 1: 1, gave the required product as an oil (6.7%). Rf ($SiO_2$/methyl acetate : EtOH, 10 : 1) = 0.36; $\nu$ ($CHCl_3$) 1800, 1745, 1700 $cm^{-1}$; $\delta$($CDCl_3$) 2.1 (3H, s, N-$CH_3$), 2.98 (1H, d, J 17 Hz, 6β-CH, partially obscured by d at $\delta$ 3.1), 3.1 (2H, d, J 7 Hz, 9-$CH_2$), 3.38 (2H, s, $CH_2$-pyridyl), 3.42 (1H, dd, J 17, and 3 Hz, 6α-CH), 4.72 (1H, t, J 7 Hz, 8-CH), 5.08 (1H, bs, 3-CH), 5.17 (2H, s, $CH_2C_6H_5$), 5.63 (1H, d, J 3 Hz, 5-CH), 7.28 (2H, m, pyridyl 4,5-H), 7.59 (1H, m, pyridyl 6-H), 7.31 (5H, s, $C_6H_5$), 8.46 (1H, m, pyridyl 2-H).

Example 24

9-N-Methyl-N-(pyrid-3-ylmethyl)aminodeoxyclavulanic acid

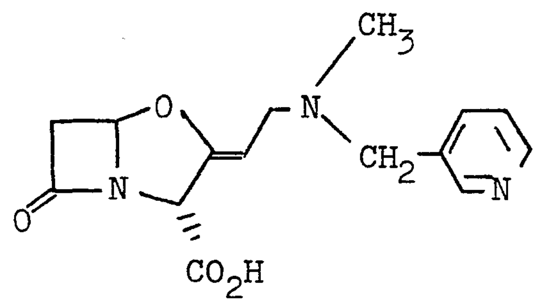

Benzyl 9-N-methyl-N-(pyrid-3-ylmethyl)aminodeoxyclavulanate (600 mg) was hydrogenolysed at ambient pressure in ethanol (50 ml) in the presence of 10 % palladium on carbon (200 mg) for 1 hour. The catalyst was removed by filtration and the filtrate evaporated to a gum which on trituration with acetone/ether gave the tertiary amine zwitterion as an off-white solid (28%). Rf ($SiO_2$/n-butanol: propan-2-ol : water, 7:7:6) = 0.16; ν (KBr) 3420, 1787, 1688, 1615 $cm^{-1}$; δ ($D_2O$) 2.75 (3H, s, N-$CH_3$), 3.05 (1H, d, J 17 Hz, 6β-CH), 3.56 (1H, dd, J 17 and 3 Hz, 6α-CH), 3.82 (2H, d, J 8 Hz, 9-$CH_2$), 4.33 (2H, $CH_2$-pyridyl), 4.82 (1H, dt, J 8 and 2 Hz, 8-CH), 4.99 (1H, bs, 3-CH), 5.72 (1H, d, J 3 Hz, 5-CH), 7.0 (1H, m, pyridyl 5-H), 7.41 (1H, m, pyridyl 4-H), 8.04 (2H, m, pyridyl 2,6-H).

Example 25

Benzyl 9-N-(furfur-2′-yl methyl)-N-(2″-hydroxyethyl)-aminodeoxyclavulanate

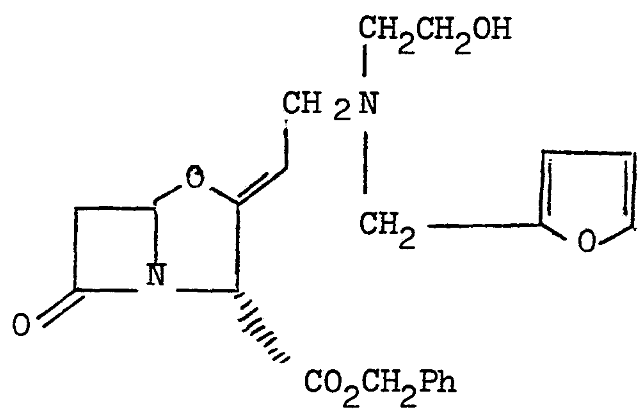

Benzyl dichloroacetyl clavulanate (8.0 g) in dimethylformamide (100 mls) was treated with 2-(furfur-2′-yl methyl)-aminoethanol (5.3 g) at room temperature for 30 minutes. The solution was poured into ethyl acetate and extracted with brine, dried ($MgSO_4$) and evaporated to a yellow oil. This was chromatographed on silica gel (elution: petrol/ethyl acetate 1:1 grading to petrol/methyl acetate 1:1) to give the title ester as an oil, 1.2 g(15%).

I.R. ($CHCl_3$) 3600-3300, 1798, 1730 and 1695 (shoulder)$cm^{-1}$.

N.M.R. ($CDCl_3$) 2.53 (2H, t, *J*, 6 Hz, $CH_2C\underline{H}_2$-N), 2.70 (1H, bs, exch. $D_2O$, $O\underline{H}$), 2.98 (1H, d, *J* 17 Hz, 6β-$C\underline{H}$), 3.19 (2H, d, *J* 7 Hz, 9-$C\underline{H}_2$), 3.43 (1H, dd, *J* 17 and 3 Hz 6α-$C\underline{H}$), 3.50 (2H, t, *J* 6 Hz, $C\underline{H}_2OH$), 3.54 (2H, s, $C\underline{H}_2$-furyl), 4.69 (1H, bt, *J* 7 Hz, 8-$C\underline{H}$), 5.08 (1H, s, 3-$C\underline{H}$), 5.19 (2H, s,-$CO_2C\underline{H}_2$-), 5.64 (1H, d, *J* 3Hz, 5-$C\underline{H}$), 6.12 (1H, m, furyl 4-$C\underline{H}$), 6.28 (1H, m, furyl 3-$C\underline{H}$) and 7.20 (6H, m, $C_6\underline{H}_5$ and furyl 5-$C\underline{H}$).

Example 26

Benzyloxycarbonylmethyl 9-N,N-bis(pyrid-3-ylmethyl) aminodeoxyclavulanate

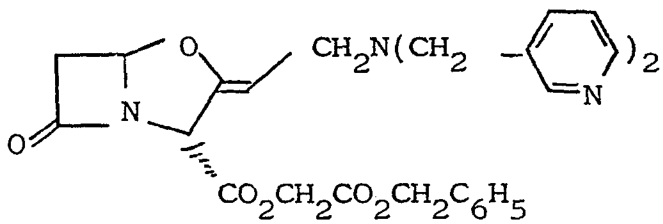

Benzyloxycarbonylmethyl 9-O-dichloroacetylclavulanate (4.58 g; 10 mM) at 0°C in dry dimethylformamide (50 $cm^3$) was treated with 1.9 equivalents of bis-(pyrid-3-ylmethyl)amine and stirred at 0° for 4 hours. The reaction mixture was poured into ethylacetate (250 $cm^3$) and washed with water (6 x 100 $cm^3$) and saturated brine (6 x 100 $cm^3$), dried (anhydrous magnesium sulphate) and evaporated to an oil. This oil was chromatographed on silica eluting with ethyl acetate/cyclohexane, 1:4 grading to 1:2. Fractions were collected containing the title compound, combined fractions were evaporated to yield an oil (1.0 g, 19%), Rf ($SiO_2$/ethanol : chloroform; 1:4) = 0.87, $\nu$(film) 1805, 1755, 1695 $cm^{-1}$. The proton magnetic resonance spectrum was consistent with the title compound.

Example 27

9-N-(Pyrid-3′-ylmethyl)aminodeoxyclavulanic acid

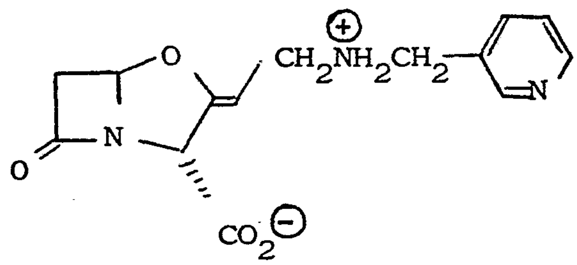

Benzyloxycarbonylmethyl 9-N,N-bis(pyrid-3′-ylmethyl)aminodeoxyclavulanate (0.95 g; 1.8 mM) in aqueous tetrahydrofuran (50 $cm^3$) was hydrogenolysed at atmospheric pressure in the presence of palladium on carbon; 10% Pd (which had been prehydrogenated for 20 minutes) for 5 hours. The catalyst was filtered off and washed with a little aqueous tetrahydrofuran. Palladium on carbon (0.3 g) was added and hydrogenolysis continued for 16 hours. The catalyst was filtered off and washed with aqueous tetrahydrofuran, the filtrate was evaporated to a foam. This foam was chromatographed on cellulose eluting with butanol - propanol - water (4:4:1). Fractions containing the title compound were collected and evaporated to yield a crystalline solid. $\nu$ (Nujol) 1812, 1695, 1615, 1592 $cm^{-1}$.

Example 28

Benzyl-9-N-(2'-methylallyl)-N-(pyrid-2''-ylmethyl)amino-deoxyclavulanate

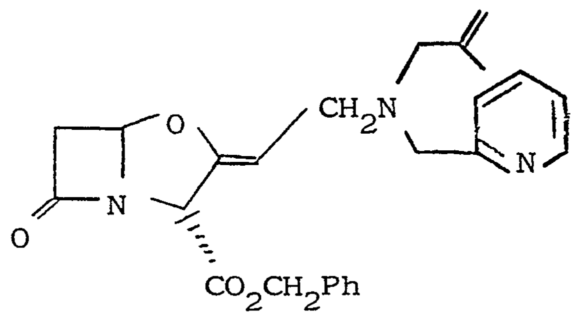

Benzyl dichloroacetylclavulanate (8.0 g, 20 mmol) in dimethylformamide (100mls) at $0^{o}C$ was treated dropwise with N-(2-methylallyl)-N-(pyrid-2'-yl-methyl)amine (6.16 g, 38 mmol) in dimethylformamide (25 ml). After 1½ hours at $0^{o}C$ and 2½ hours at room temperature the reaction mixture was poured into water and extracted with ethyl acetate. The organic phase was washed with water (3 x), brine, dried ($MgSO_4$) and evaporated *in vacuo* to an oil. Chromatography on silica gel (elution : petroleum ether/ ethyl acetate 4/1 grading to 2/3) afforded the title compound as an oil, 1.22 g (14%).

I.R. (film) 1803, 1755, 1698, 1300 and 900 $cm^{-1}$.

NMR ($CDCl_3$) 1.73 (3H, s, $-CH_3$), 2.89 (2H, s, $-CH_2-$), 2.92 (1H, d, J 17 Hz, 6β-CH), 3.17 (2H, d, J 8 Hz, 9-$CH_2$), 3.38 (1H, dd, J 17 and 3 Hz, 6α-CH), 3.63 (2H, s, $-CH_2-$), 4.75 (1H, t, J 8 Hz, 8-CH), 4.86 (2H, dm, $=CH_2$), 5.04 (1H, s, 3-CH), 5.14 (2H, s, $-CO_2CH_2-$), 5.58 (1H, d, J 3 Hz, 5-CH), 7.00 - 7.70 (8H, m, $C_6H_5$ and pyridyl-3H, 4H and 5H) and 8.47 (1H, m, pyridyl-6H).

Example 29

9-N-(2'-Methylallyl)-N-(quinol-3''-ylmethyl)aminodeoxyclavulanic acid

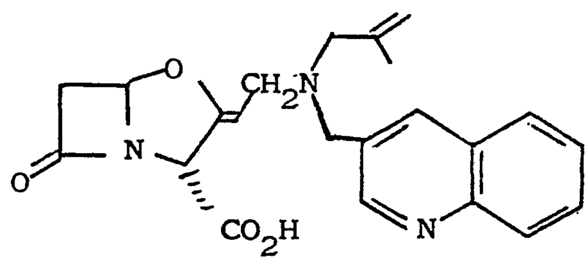

10% Pd/C (0.060 g) in ethanol (10 ml) was prehydrogenated for 10 mins. Benzyl 9-N-(2'-methylallyl)-N-(quinol-3''-ylmethyl)aminodeoxyclavulanate (0.170 g, 0.35 mmol) in ethanol (5 ml) was added and hydrogenation was carried out for 2 hours. Catalyst was filtered off and solvent removed *in vacuo* to give a yellow oil. Chromatography on silica gel (elution :THF/EtOH/$H_2O$ 20/1/1) afforded the title product as an oil (0.040 g, 29%).

I.R. (film) 3600-3200, 1795, 1695 and 1620 $cm^{-1}$.

Example 30

9-Pyrid-4-ylmethylaminodeoxyclavulanic Acid

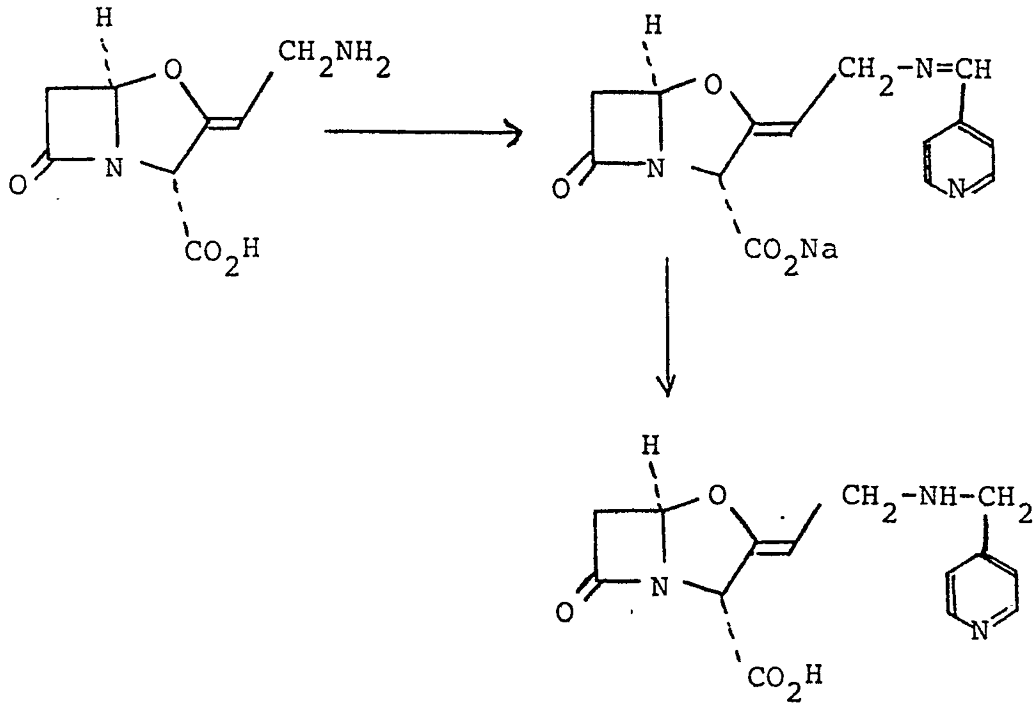

9-Aminodeoxyclavulanic acid (200 mg) was dissolved in water (10 ml) and pyridyl-4-aldehyde (0.43 g, 4x excess) in tetrahydrofuran (10 ml) added. The pH during addition was maintained at pH 8.0 on the pH-Stat by the addition of 1M NaOH solution. The reaction became very slow when 0.92 ml NaOH had been taken up. At this point the solution contained the Schiffs base.

The NaOH burette was replaced with a 1M HCl burette, and portions of $NaBH_4$ (20 mg each) added until each successive portion did not cause a large change in pH. The HCl burette was used to maintain the reaction mixture at 9.0 - 9.5. Tlc (butanol - isopropanol - water 7:7:6) then showed complete conversion to a product whose Rf value was less than the starting 9-aminodeoxyclavulanic acid.

The solution was evaporated to dryness and triturated with acetone. The insoluble material (300 mg) was subjected to silica gel chromatography using butanol - ethanol - water 4:4:1 was the eluent. After a non-β-lactam containing component had eluted, fractions containing the required product (by tlc) were collected, evaporated, triturated with acetone and filtered off, to give 9-pyrid-4-ylmethylaminodeoxyclavulanic acid (40 mg) as a pale cream solid.

I.r. $cm^{-1}$ 1698, 1802, 2400 to 3600 (broad with fine structure). (Nujol).

N.m.r. ν ($D_2O$) 3.00(1H, d, J 17 Hz, 6β-CH), 3.49(1H, dd, J 3 and 17 Hz, 6α-CH), 3.58(2H, d, J 7Hz, 9-$CH_2$), 4.06(2H, s, $CH_2$-pyridyl), 4.75(1H, t, partly obscured by HOD, 8-CH), 4.90(1H, s, 3-CH), 5.65(1H, d, J 3Hz, 5-CH), 7.37(2H, d, J 6Hz, 3,5,pyridyl H), 8.47(2H, d, J 6 Hz, 2,6, pyridyl H).

Example 31

9-Isothiazol-5-yl-aminodeoxyclavulanic Acid

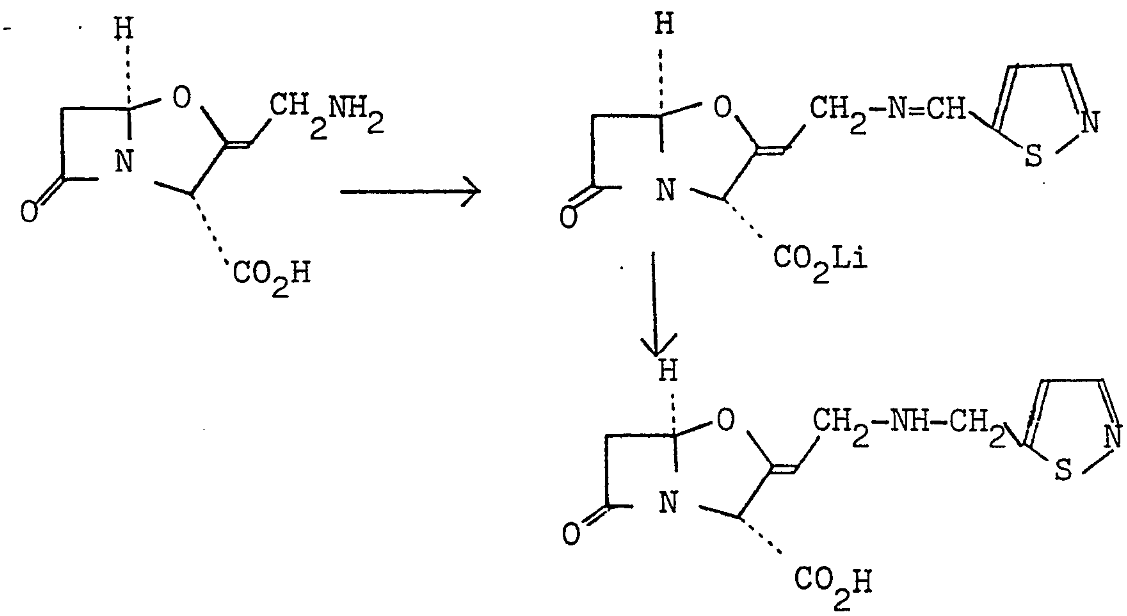

To a stirred solution of 9-aminodeoxyclavulanic acid (0.2g) in water (10ml) and tetrahydrofuran (10ml) was added isothiazole-5-carboxaldehyde (0.5ml). The pH was maintained constant at 8.3 by the addition of lithium hydroxide solution (1M). The theoretical amount was taken up (1ml). At this point the solution contained the Schiffs base. The pH of the solution was increased to 9, and sodium borohydride (0.2g) was added in small portions during 15 mins, maintaining the pH of the solution between 9 and 10 (mostly about 9.2) by the simultaneous addition of 1M HCl. T.l.c. then showed that a new zone with Rf slightly higher than the primary amine had appeared ($SiO_2$ - butanol/isopropanol/water - 7:7:6). The reaction mixture was evaporated to dryness, and extracted with ethyl acetate (2 x 25ml). The insoluble material was dissolved in water (about 2ml) and subjected to column chromatography on silica gel using butanol/isopropanol/water, 7:7:6, as the elution solvent. Fractions containing the required product (by t.l.c) were combined and evaporated to a colourless crystalline solid, which

was triturated with acetone (20ml), filtered off, washed with ether and dried in air, to yield 9-isothiazol-5-ylaminodeoxyclavulanic acid (0.18g).

I.r (nujol mull) 2500 - 3800 (broad, with fine structure), 1808, 1693, 1615 and 1575 $cm^{-1}$.

Demonstration of Activity

The antibacterial activity *in vitro* of compounds of the invention is illustrated by the results shown in Table 1.

The minimum inhibitory concentration (MIC) of ampicillin alone and in combination with certain compounds of this invention was determined for several strains of bacteria. These synergy results are shown in Table 2.

Mice infected intraperitoneally with *E. coli* E96 were treated sub-cutaneously with amoxycillin, amoxycillin and a compound of the invention or cefazolin at 1 and 5 hours post-infection. The $CD_{50}$ values are shown in Table 3.

TABLE I

| Compound of Example | MIC (μg/ml) | | |
| --- | --- | --- | --- |
| | Staph. aureus Russel | Klebsiella aerogenes E70 | E. coli JT 39 |
| 10 | 2.0 | 250 | 31.2 |
| 11 | 125 | >500 | >500 |
| 13 | 4.0 | 250 | 62.5 |
| 14 | 62.5 | >500 | >500 |
| 17 | 8 | 500 | 15.6 |
| 21 | 2 | 125 | 31.2 |
| 24 | 8 | 125 | 15.6 |
| 29 | 25 | >200 | >200 |
| 30 | 16 | 500 | 125 |
| 31 | 16 | 250 | 125 |

TABLE 2

| Compound of Example | Conc. (μg/ml) | MIC Ampicillin (μg/ml) | | |
|---|---|---|---|---|
| | | Staph.aureus Russell | Klebsiella aerogenes E 70 | E. coli JT 39 |
| 10 | 5 | (<0.01) | 1.6 | 2.0 |
| | 1 | 0.04 | 3.1 | 8.0 |
| | 0 | 62.5 | >500 | >500 |
| 11 | 20 | ca.0.1 | 25 | 250 |
| | 5 | 0.4 | 25 | >500 |
| | 0 | 125 | 250 | 2000 |
| 13 | 5 | Inhib. | 6.25 | 4.0 |
| | 1 | < 0.01 | 6.25 | 16.0 |
| | 0 | 500 | 1000 | >2000 |
| 14 | 20 | 0.04 | 50 | >500 |
| | 5 | 0.3 | 100 | >500 |
| | 0 | 250 | 500 | 2000 |
| 17 | 5 | (0.02) | 3.12 | 4 |
| | 1 | 0.3 | 12.5 | 15.6 |
| | 0 | 1000 | 1000 | >2000 |
| 21 | 5 | Inhib. | (0.4) | (2) |
| | 1 | Inhib. | 3.1 | 4 |
| | 0 | 500 | 500 | 2000 |
| 24 | 5 | (≤0.01) | 3.12 | 2 |
| | 1 | 0.3 | 3.12 | 8 |
| | 0 | 1000 | 1000 | >2000 |
| 29 | 5 | (0.16) | 50 | 250 |
| | 1 | 0.3 | 100 | >500 |
| | 0 | 500 | 500 | >2000 |
| 30 | 5 | 0.08 | 3.1 | 4.0 |
| | 1 | 0.6 | 0.6 | 31.2 |
| | 0 | 500 | 125 | 2000 |

TABLE 2 continued....

| Compound of Example | Conc. (μg/ml) | MIC Ampicillin (μg/ml) | | |
|---|---|---|---|---|
| | | Staph.aureus Russell | Klebsiella aerogenes E 70 | E. coli JT 39 |
| 31 | 5 | 0.04 | 3.1 | 8 |
| | 1 | 0.6 | 6.2 | 8 |
| | 0 | 500 | 500 | 2000 |

"Inhib." = some inhibition by compound alone
"(    )" = inhibition of control observed

TABLE 3

| | Compound of Example | Dosage (mg/kg) | $CD_{50}$ (mg/kg) |
|---|---|---|---|
| Amoxycillin | - | - | >1000 x 2 |
| | 17 | 5 | 10 x 2 |
| | 21 | 5 | 12 x 2 |
| Cefazolin | - | - | 14 x 2 |

Composition

50 mg of the compound of Example 17 was dissolved in 2.5 ml sterile water for injection BP to provide a solution suitable for injection.

What we claim is :

1. A compound of the formula (II) :

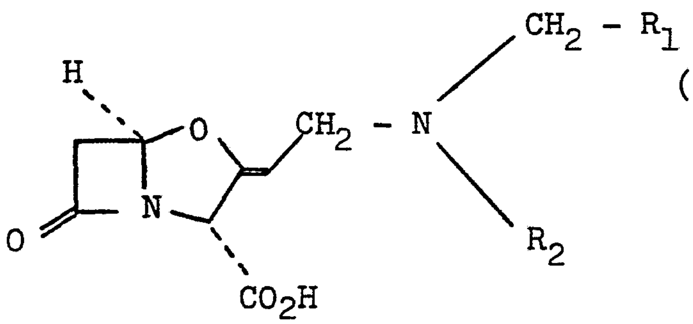

(II)

or an ester or an acid addition salt of an ester thereof wherein $R_1$ is an optionally substituted aromatic monocyclic or bicyclic heterocyclic C-bonded group and $R_2$ is a group $CH_2R_1$, a hydrogen atom, a $C_{1-6}$ alkyl or hydroxyalkyl group,an optionally substituted benzyl group, or a group of the sub-formula (a) :

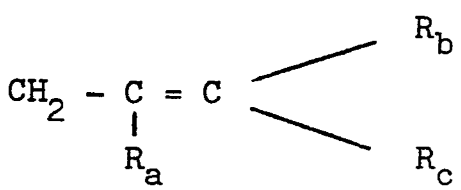

(a)

where $R_a$ is a $C_{1-3}$ alkyl group, and $R_b$ and $R_c$ are independently hydrogen, $C_{1-3}$ alkyl or phenyl.

2. A compound according to claim 1 wherein $R_1$ is a group of the sub-formula (i) :

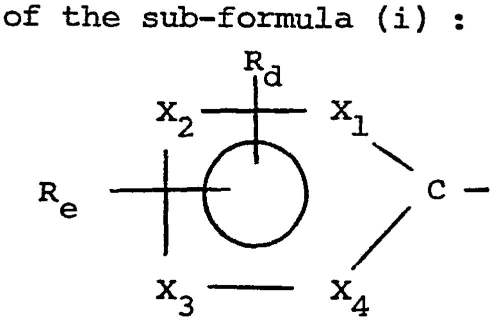

(i)

wherein one of $X_1$, $X_2$, $X_3$ and $X_4$ is -CHN-, -O- , -S- or $-NR_f-$ where $R_f$ is H or lower alkyl, and the others are selected from -N- and -CH-; $R_d$ is hydrogen; and $R_e$ is hydrogen, lower alkyl, CN, $CO_2H$, esterified $CO_2H$ or $NO_2$; or $R_d$ and $R_e$ together form the residue of an optionally substituted benzene ring.

3. A compound according to claim 1 or claim 2 wherein the heterocyclic group contains up to 3 heteroatoms i in the ring system.

4. A compound according to any one of claims 1 to 3 wherein $R_1$ is a furyl, thienyl, pyrrolyl, N-methylpyrrolyl, pyridyl, indolyl, oxazolyl, thiazolyl or quinolyl group.

5. A compound according to any one of claims 1 to 4 wherein $R_2$ is a hydrogen atom or a methyl, ethyl, propyl, butyl, hydroxymethyl,hydroxyethyl or hydroxypropyl group.

6. A compound according to claim 5 wherein $R_2$ is a hydrogen atom.

7. A compound according to any one of claims 1 to 4 wherein $R_2$ is a group of the sub-formula (b) :

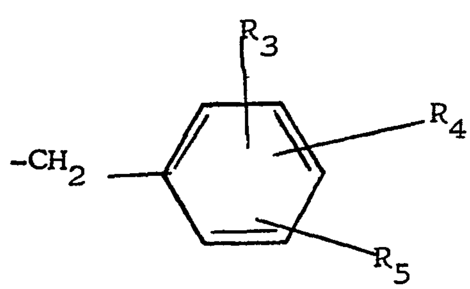

(b)

wherein $R_3$ is a hydrogen, fluorine, chlorine or bromine atom or an alkyl group of 1-3 carbon atoms, an

alkoxyl group of 1-3 carbon atoms, an acyloxy group of 1-3 carbon atoms, a hydroxyl group or an alkoxycarbonyl group containing 1-3 carbon atoms in the alkoxy part; $R_4$ is a hydrogen, fluorine or chlorine atom or an alkyl group of 1-3 carbon atoms, an alkoxy group of 1-3 carbon atoms or an acyloxy group of 1-3 carbon atoms; $R_5$ is a hydrogen, fluorine or chlorine atom or an alkyl group of 1-3 carbon atoms or an alkoxyl group of 1-3 carbon atoms.

8. A compound according to claim 7 wherein the group of the sub-formula (b) is a benzyl, p-methylbenzyl, p-methoxybenzyl, p-fluorobenzyl or p-chlorobenzyl group.

9. A compound according to any one of claims 1 to 4 wherein $R_2$ is a 2-methylallyl or 2-methyl-3-phenylallyl group.

10. A compound according to any one of claims 1 to 4 wherein $R_2$ is a group $CH_2R_1$ where $R_1$ is as defined in any of claims 1 to 4.

11. A compound of the formula (III):

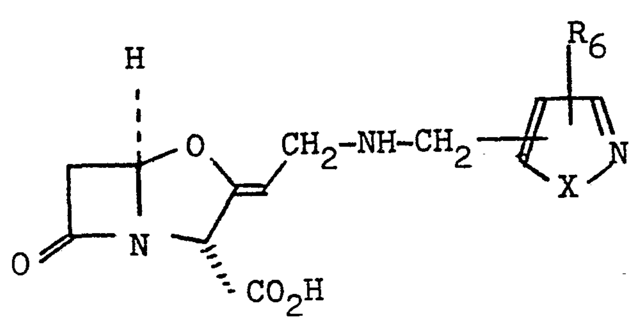

(III)

wherein X is a sulphur atom or an oxygen atom and $R_6$ is a hydrogen atom or an alkyl group of 1-3 carbon atoms.

12. A compound of the formula (IV), (V), (VI) (VII), (XVI) or (XVII) :

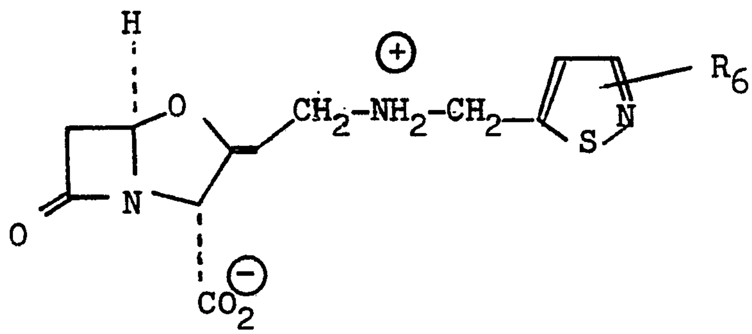

(IV)

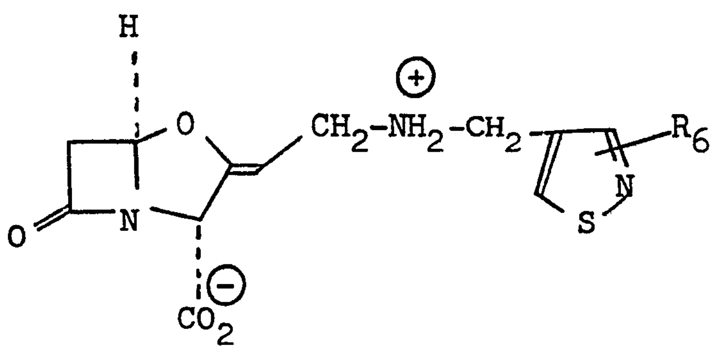

(V)

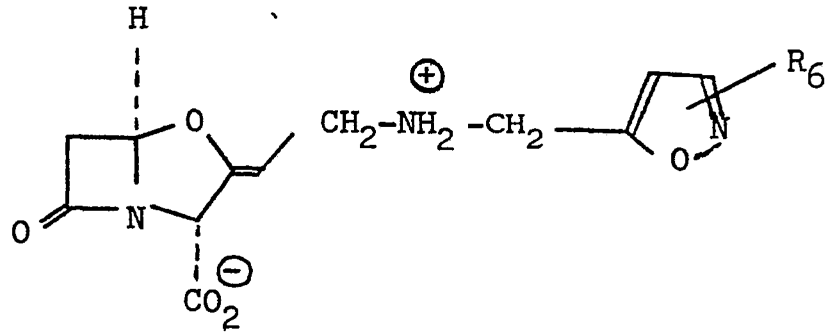

(VI)

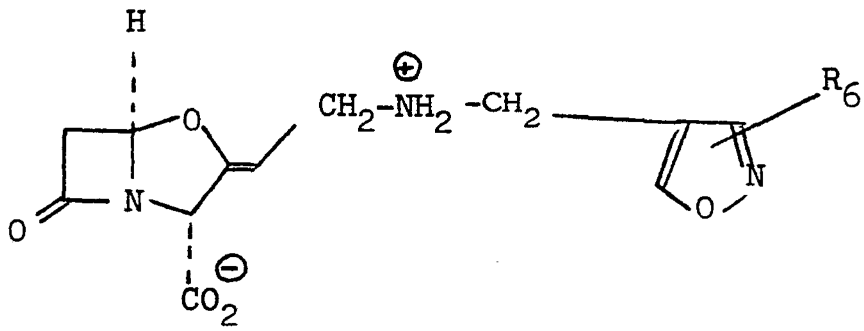

(VII)

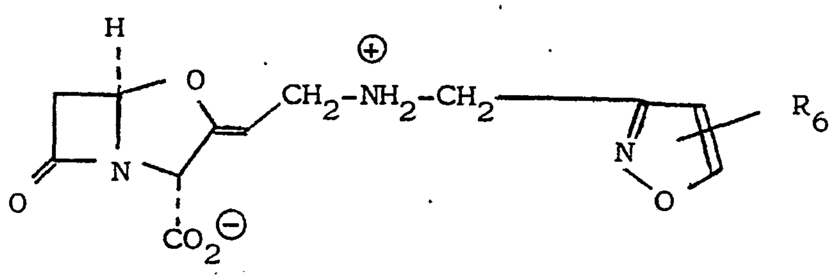

(XVI)

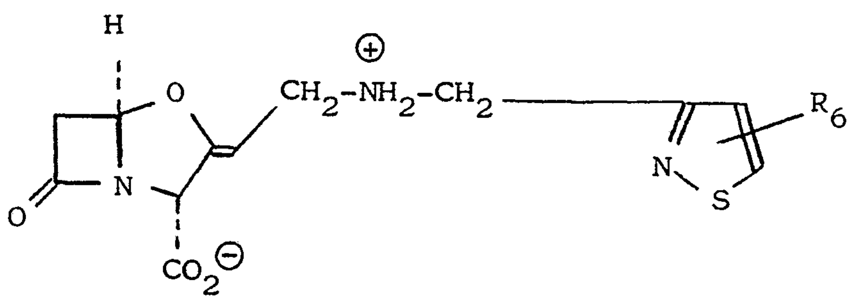

(XVII)

wherein $R_6$ is as defined in claim 11.

13. A compound according to claim 11 or claim 12 wherein $R_6$ is a hydrogen atom or a methyl group.

14. A compound of the formula (VIII) or (IX):

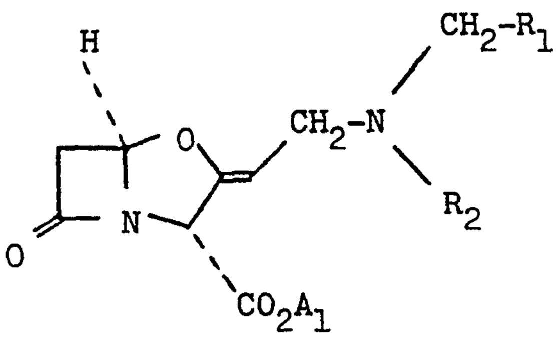

(VIII)

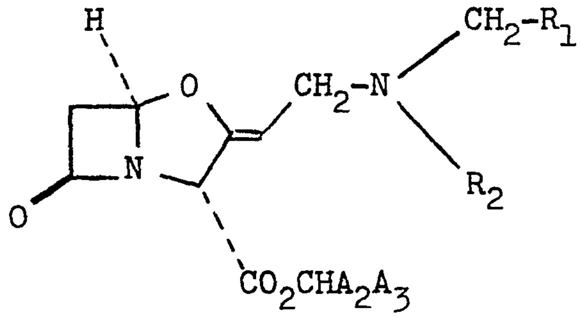

(IX)

wherein $R_1$ and $R_2$ are as defined in any of claims 1 to 10; $A_1$ is an alkyl group of 1-6 carbon atoms optionally substituted by an alkoxyl or acyloxyl group of 1 - 7 carbon atoms; $A_2$ is an alkenyl group of up to 5 carbon atoms or is a phenyl group optionally substituted by a fluorine, chlorine, bromine, nitro or alkyl or alkoxyl of up to 4 carbon atoms; and $A_3$ is a hydrogen atom, an alkyl group of up to 4 carbon atoms or a phenyl group optionally substituted by a fluorine, chlorine, bromine, nitro or alkyl or alkoxyl of up to 4 carbon atoms.

15. An ester of a compound of the formula (II) which is a methyl, ethyl, n-propyl, n-butyl, allyl, $CH_2C{\equiv}CH$, methoxy-methyl, acetoxymethyl, priopionoxymethyl, pivaloyloxymethyl, ethoxycarbonyloxymethyl, methoxycarbonyloxyethyl, ethoxycarbonyloxyethyl, dimethyoxyphthalidyl, benzyl, methoxybenzyl, ethoxybenzyl, nitrobenzyl or chlorobenzyl ester.

16. A compound according to claim 14 wherein $A_1$ is a methyl, methoxymethyl, acetoxymethyl, acetoxyethyl, phthalidyl, ethoxycarbonyloxy-methyl, or α-ethoxycarbonyloxyethyl group.

17. A compound according to claim 14 wherein $A_2$ is a phenyl, p-nitrophenyl or p-methoxyphenyl group.

18. A compound according to claim 14 or claim 17 wherein $A_3$ is a hydrogen atom.

19. An ester according to any one of claims 1 to 10 or 14 to 18 which is in the form of an acid addition salt with a pharmaceutically acceptable acid.

20. A compound according to any one of claims 1 to 10 which is in the form of a zwitterion.

21. A process for the preparation of a compound according to claim 1, which process comprises the reaction of an ester of the compound of the formula (X):

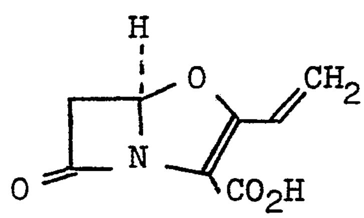

(X)

with a compound of the formula (XI):

$$HN(R_2)CH_2R_1 \qquad (XI)$$

wherein $R_1$ and $R_2$ are as defined in relation to formula (II) except that $R_2$ is not a hydrogen atom, and thereafter, if desired, performing one or more of the following reactions :

(a) when it is desired to form a compound (II) where $R_2$ is H, converting a compound where $R_2$ is a group (a), $CH_2R_1$ or benzyl to the corresponding compound where $R_2$ is H by hydrogenation;

(b) converting the ester of the compound of the formula (II) to an acid addition salt, the free acid or an alternative ester or acid addition salt thereof.

22. A process according to claim 21 for the preparation of a compound (II) where $R_2$ is hydrogen, wherein step (a) comprises the hydrogenation of a compound (II) wherein $R_2$ is benzyl, a group $CH_2R_1$ where $R_1$ is as defined in relation to formula (II), 2-methylallyl or 2-methyl-3-phenylallyl.

23. A process according to claim 22 for the preparation of a zwitterionic secondary amine of the formula (II), which process comprises the hydrogenation of a hydrogenolysable ester of a compound of the formula (II) where $R_2$ is a hydrogenolysable group.

24. A process for the preparation of a compound of the formula (II) wherein $R_2$ is a hydrogen atom, which process comprises the reduction with a water-soluble complex hydride of a salt of a compound of the formula (XII):

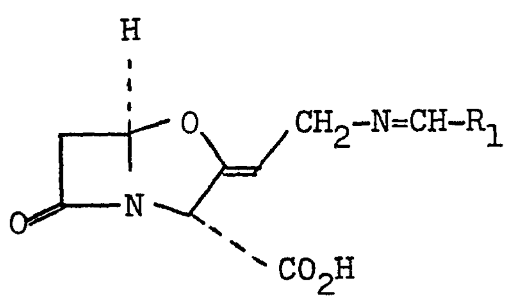

(XII)

wherein $R_1$ is as defined in relation to formula (II).

25. A process according to claim 24 wherein the complex hydride is lithium borohydride, sodium borohydride, potassium borohydride or sodium cyanoborohydride.

26. A process according to claim 24 or claim 25, which process comprises the reduction of a salt of a compound of the formula (XIII) :

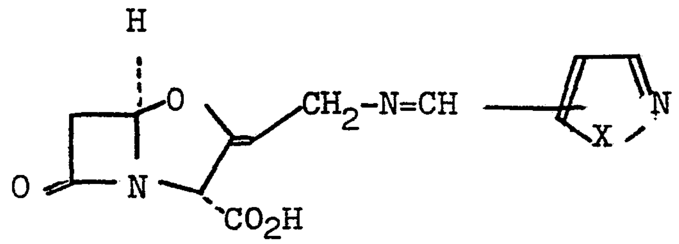

(XIII)

wherein X and $R_6$ are as defined in claim 10.

27. A process for the preparation of an acid addition salt of an ester of a compound of the formula (II) as defined in claim 1, which process comprises the hydrogenation of a corresponding ester of a compound of the formula (XIV) :

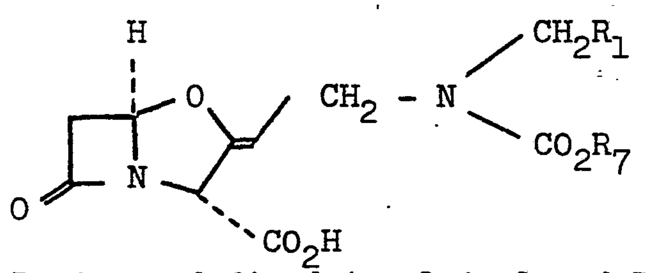

(XIV)

wherein $R_1$ is as defined in claim 1 and $R_7$ is a group of the sub-formula (b) as defined in claim 1, and thereafter converting the product to the acid addition salt.

28. A process according to claim 27 wherein $R_7$ is a benzyl group.

29. A process according to claim 27 or claim 28 wherein a $C_{1-4}$ alkyl ester is used.

30. A process for the preparation of a compound of the formula (II) as defined in claim 1, which process comprises the hydrogenation of a hydrogenolysable ester of the compound of the formula (II).

31. A process for the preparation of a compound of the formula (II) as defined in claim 1, which process comprises the mild basic hydrolysis of a corresponding ester.

32. A pharmaceutical composition comprising a compound according to any one of claims 1 to 20 and a pharmaceutically acceptable carrier.

33. A composition according to claim 32 adapted for administration by injection or infusion.

34. A composition according to claim 32 in unit dose form adapted for oral administration.

35. A composition according to claim 32 adapted for topical administration.

36. A composition according to any one of claims 32 to 35 which also comprises a penicillin or cephalosporin.

37. A composition according to claim 36 comprising ampicillin, amoxycillin, carbenicillin or ticarcillin.

38. A compound according to claim 1 produced in any of Examples 1 to 31 herein.

39. A compound according to claim 1 when prepared by the process of claim 21.

40. A compound according to claim 1 when prepared by the process of claim 24.

European Patent Office

# EUROPEAN SEARCH REPORT

0005014
Application number
EP 79 300 515.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.²) |
|---|---|---|---|
| D | DE - A - 2 646 003 (BEECHAM) * claim 30 * -- | 21 | C 07 D 498/04<br>A 61 K 31/42 |
| P,A | DE - A - 2 817 085 (BEECHAM) -- | | |
| P,A | DE - A - 2 818 309 (BEECHAM) ---- | | |

TECHNICAL FIELDS SEARCHED (Int.Cl.²)

A 61 K 31/42
C 07 D 498/04

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 10-07-1979 | FROELICH |

EPO Form 1503.1 06.78